(19) Europäisches Patentamt / European Patent Office / Office européen des brevets

(11) **EP 3 406 623 A1**

(12) **EUROPEAN PATENT APPLICATION**
published in accordance with Art. 153(4) EPC

(43) Date of publication:
**28.11.2018 Bulletin 2018/48**

(21) Application number: **17741374.7**

(22) Date of filing: **17.01.2017**

(51) Int Cl.:
*C07K 1/18* (2006.01)    *B01D 15/36* (2006.01)
*B01J 20/281* (2006.01)    *G01N 30/00* (2006.01)
*G01N 30/88* (2006.01)

(86) International application number:
**PCT/JP2017/001364**

(87) International publication number:
**WO 2017/126496 (27.07.2017 Gazette 2017/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.01.2016 JP 2016011051**

(71) Applicant: **Asahi Kasei Medical Co., Ltd.**
**Chiyoda-ku**
**Tokyo 101-8101 (JP)**

(72) Inventors:
- **YOKOYAMA, Yoshiro**
**Tokyo 101-8101 (JP)**
- **TANIGUCHI, Hiroki**
**Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **PROTEIN PURIFICATION METHOD**

(57) The present invention provides a method for purifying a protein, comprising: providing a solution containing a monomer and aggregates of the protein of interest; a purification step of removing the aggregates of the protein of interest using a cation-exchange chromatographic support media to obtain a purified solution of the monomer, the cation-exchange chromatographic support media comprising at least one type of weak cation-exchange group and having a cation-exchange group density higher than 30 mmol/L; and a virus removal step of removing viruses from the purified solution using a virus removal membrane having a virus logarithmic reduction value of 3 or more.

Fig. 11

# EP 3 406 623 A1

**Description**

Technical Field

The present invention relates to a method for purifying a protein.

Background Art

Immunoglobulins (antibodies) are physiologically active substances that are responsible for immune response. In recent years, their use values have been increasing in applications such as pharmaceutical products, diagnostic drugs, and materials for separation and purification of corresponding antigenic proteins. The antibodies are obtained from the blood of immunized animals, cell culture solutions of cells possessing the ability to produce antibodies, or ascitic fluid culture solutions of animals. However, such antibody-containing blood or culture solutions contain proteins other than the antibodies, or complicated foreign components derived from stock solutions used in the cell culture. The separation and purification of the antibodies from these impurity components usually require a complicated and time-consuming operation.

Liquid chromatography is important for antibody separation and purification. Examples of chromatography approaches for antibody separation include gel filtration chromatography, affinity chromatography, ion-exchange chromatography, and reverse-phase chromatography. Antibodies are separated and purified by combining these approaches.

The ion-exchange chromatography is a method which involves using ion-exchange groups on the surface of an adsorbent as a stationary phase and reversibly adsorbing thereon counterions present in a mobile phase for separation. For example, beads or a membrane (e.g., flat membranes and hollow fiber) is adopted as the shape of the adsorbent. These substrates bound with cation-exchange groups or anion-exchange groups are commercially available as adsorbents.

Purification using the adsorbent having cation-exchange groups is generally performed by contacting an antibody solution having a low salt concentration with the adsorbent so that antibodies are adsorbed thereon, and eluting the adsorbed physiologically active substances by increasing the salt concentration of a mobile phase. The purification of a substance of interest in a flow-through manner described below has also been proposed as a more favorable method.

The flow-through manner is a manner of a purification method that selectively adsorbs impurities rather than the substance of interest onto an adsorbent. Therefore, this approach leads to a saving in buffer solution and simplification of steps, as compared with conventional methods using adsorption and elution. Also, it is considered that the advantages of the flow-through purification can be further exploited if an antibody solution can be processed at a high flow rate.

A cation-exchange step is often aimed at separating antibody monomers from aggregates such as antibody dimers. However, the antibody monomers and the antibody aggregates have almost equal isoelectric points. Therefore, the separation of antibody monomers from antibody dimers is particularly difficult for the flow-through purification and requires the detailed design of a cation-exchanger, including the density of cation-exchange groups. In addition, each antibody has distinctive properties. Therefore, the same design is not always optimal for all antibodies even if the design is detailed. A cation-exchange group density suitable for each antibody may be present.

Patent Literature 1 discloses a multimodal chromatographic resin for use in flow-through purification, comprising a resin bound with low-molecular compounds having aromatic and weak cation-exchange groups.

Patent Literature 2 discloses a chromatographic support media comprising silica beads bound with copolymers having weak cation-exchange groups.

Patent Literature 3 discloses a chromatographic support media suitable for flow-through purification in which monomers having strong cation-exchange groups and uncharged (neutral) monomers are graft-polymerized onto a support media.

In recent years, human blood-derived plasma derivatives as well as biopharmaceuticals have required an approach of improving viral safety. Therefore, pharmaceutical manufacturers have discussed the introduction of a virus removal/inactivation step into a manufacturing process. Among others, a method for removing viruses by filtration using a virus removal membrane is an effective method that can reduce viruses without denaturing useful proteins.

Particularly as to parvovirus among viruses, there have been reports on cases of infection by human parvovirus B19 in the field of plasma derivatives and the contamination of CHO (Chinese hamster ovary) cells with mouse parvovirus in the field of biopharmaceuticals. The parvovirus, which is a small virus having no envelope, is physicochemically stable and is resistant to heating, low pH, and treatment with chemicals in an inactivation step generally performed in a manufacturing process of pharmaceutical products. Therefore, there is a growing need for parvovirus removal using a virus removal membrane as a method for removing viruses under the mechanism of action different from that of the inactivation method.

Citation List

Patent Literature

Patent Literature 1: Japanese Patent No. 4776615
Patent Literature 2: Japanese Patent No. 5234727
Patent Literature 3: Japanese Patent Laid-Open No. 2013-189427

Summary of Invention

Technical Problem

In the practice of manufacturing pharmaceutical products, there is a demand for a virus removal membrane having a high property of removing small viruses (e.g., parvovirus) similar in size to useful proteins, and high protein filtration efficiency. Requirements for virus removal membranes get stricter every year.

In response to this, the total amount of viruses loaded on a virus removal membrane (the amount of viruses spiked to a pharmaceutical protein or the total filtration volume) is increased in the evaluation tests of virus removal membranes that indicate the ability of a virus removal step in a manufacturing process. Thus, conditions for passing the evaluation tests of virus removal membranes get stricter every year.

However, it has heretofore been difficult to suppress reduction in permeate flux in a virus removal step while maintaining high virus removal performance. Thus, an object of the present invention is to provide a method for purifying a protein that is capable of suppressing reduction in permeate flux in a virus removal step.

Solution to Problem

The present inventors have conducted studies from various angles and conducted research and development in order to attain the object. As a result, the present inventors have completed the present invention by finding that reduction in permeate flux in a virus removal step can be suppressed by removing aggregates using a cation-exchange chromatographic support media having a cation-exchange group density higher than 30 mmol/L and then removing viruses using a virus removal membrane. Although not bound by any theory, this is probably because the cation-exchange chromatographic support media removes aggregates in a solution of the protein of interest and thereby prevents the virus removal membrane to be clogged by the aggregates.

An aspect of the present invention provides a method for purifying a protein, comprising: providing a solution containing a monomer and aggregates of the protein of interest; a purification step of removing the aggregates of the protein of interest using a cation-exchange chromatographic support media to obtain a purified solution of the monomer, the cation-exchange chromatographic support media comprising at least one type of weak cation-exchange group and having a cation-exchange group density higher than 30 mmol/L; and a virus removal step of removing viruses from the purified solution using a virus removal membrane having a virus logarithmic reduction value of 3 or more.

In the aforementioned purification method, the cation-exchange chromatographic support media may comprise a membrane matrix and a copolymer immobilized on the surface of the membrane matrix, and the copolymer may comprise a (meth)acrylamide-based compound and/or a (meth)acrylate-based compound as monomer units. Monomer units other than monomer units having cation-exchange groups in the copolymer may be neutral monomers having no charge, and the neutral monomers may be a hydrophobic monomer unit and/or a hydrophilic monomer unit. The hydrophobic monomer unit may have a linear or branched alkyl group having four or more carbon atoms. The mass percentage of the hydrophobic monomer unit and/or the hydrophilic monomer unit in the copolymer may be higher than that of the monomer units having cation-exchange groups.

In the aforementioned purification method, the weak cation-exchange group may be derived from any of an acrylic acid monomer, a methacrylic acid monomer, an acrylic acid compound monomer, and a methacrylic acid compound monomer. The cation-exchange groups comprised in the cation-exchange chromatographic support media may be only weak cation-exchange groups. Alternatively, the cation-exchange groups comprised in the cation-exchange chromatographic support media may include a weak cation-exchange group and a strong cation-exchange group. The strong cation-exchange group may be a sulfonic acid group.

In the aforementioned purification method, the copolymer may be immobilized on the surface of the membrane matrix through a covalent bond.

In the aforementioned purification method, the hydrophilic monomer unit may comprise at least one of isopropylacrylamide and 2-hydroxyethyl methacrylate.

In the aforementioned purification method, the membrane matrix of the cation-exchange chromatographic support media may comprise polyethylene. The graft ratio of the copolymer graft-polymerized onto the membrane matrix may be 20 to 200%. The membrane matrix may comprise polyvinylidene fluoride. The graft ratio of the copolymer graft-polymerized onto the membrane matrix may be 5 to 100%. The copolymer substantially may have no cross-linked structure. The copolymer may comprise a monomer unit containing two or more polymerizable functional groups.

In the aforementioned purification method, the cation-exchange group density may be higher than 45 mmol/L.

In the aforementioned purification method, the virus removal membrane may comprise a primary-side surface to which the purified solution of the monomer is to be applied, and a secondary-side surface facing the primary-side surface, wherein the virus removal membrane may comprise at least a site where a pore size decreases from the primary side toward the secondary side on the cross section of the virus removal membrane.

In the aforementioned purification method, the virus removal membrane may comprise a primary-side surface to which the purified solution of the monomer is to be applied, and a secondary-side surface facing the primary-side surface, wherein a pore size may decrease and in turn increase from the primary side toward the secondary side on the cross section of the virus removal membrane.

In the aforementioned purification method, the virus removal membrane may comprise a primary-side surface to which the purified solution of the monomer is to be applied, and a secondary-side surface facing the primary-side surface, wherein a pore size may decrease and in turn become constant from the primary side toward the secondary side on the cross section of the virus removal membrane, and a most closely packed layer may be comprised near the secondary-side surface.

In the aforementioned purification method, the virus removal membrane may comprise cellulose. Alternatively, the virus removal membrane may comprise a hydrophilized synthetic polymer.

In the aforementioned purification method, no additional step may be comprised between the purification step and the virus removal step. Also, the purification step and the virus removal step may be continuously performed.

In the aforementioned purification method, the protein of interest may be an antibody. The antibody may be a monoclonal antibody. The protein of interest may be a recombinant protein.

Advantageous Effects of Invention

The method for purifying a protein according to the present invention makes it possible to suppress reduction in permeate flux in a virus removal step.

Brief Description of Drawings

[Figure 1] Figure 1 is a schematic diagram of a virus removal membrane having the shape of a hollow fiber membrane according to an embodiment of the present invention.

[Figure 2] Figure 2 is a schematic diagram of a virus capture site in a virus removal membrane having the shape of a hollow fiber membrane according to Reference Examples of the present invention.

[Figure 3] Figure 3 is a schematic diagram of a virus capture site in the virus removal membrane having the shape of a hollow fiber membrane according to the embodiment of the present invention.

[Figure 4] Figure 4 is a schematic diagram of a virus removal membrane having the shape of a flat membrane according to the embodiment of the present invention.

[Figure 5] Figure 5 is a graph of absorbance when an antibody solution according to Reference Example 1 was applied to size exclusion chromatography.

[Figure 6] Figure 6 is an enlarged view of the graph of Figure 5.

[Figure 7] Figure 7 is a table showing results of Reference Examples 1 to 29 and Reference Comparative Examples 1 to 3.

[Figure 8] Figure 8 is a table showing results of Reference Example 30.

[Figure 9] Figure 9 is a table showing results of Reference Examples 31 and 32.

[Figure 10] Figure 10 is a table showing results of Example 1, Reference Example 33, and Comparative Examples 1 and 2.

[Figure 11] Figure 11 is a graph showing results of Example 1 and Comparative Example 1.

Description of Embodiments

Hereinafter, preferred embodiments (hereinafter, referred to as "embodiments") of the present invention will be described in detail. The embodiments shown below are given for illustrating apparatuses or methods for concretizing the technical idea of this invention. The technical idea of this invention does not limit combinations of constituent members, etc., to those described below. The technical idea of this invention can be variously changed or modified within the scope of claims.

The method for purifying a protein according to an embodiment comprises: providing a solution containing a monomer and aggregates of the protein of interest; removing the aggregates of the protein of interest using a cation-exchange chromatographic support media to obtain a purified solution of the monomer, the cation-exchange chromatographic support media comprising at least one type of weak cation-exchange group and having a cation-exchange group density higher than 30 mmol/L; and removing viruses from the purified solution using a virus removal membrane having a virus logarithmic reduction value of 3 or more.

The protein of interest is, for example, an antibody protein. The aggregates of the protein of interest refer to, for example, at least any of low-order (e.g., dimer and trimer) aggregates of the protein of interest (the protein itself may be an associate such as a dimer), tetramer or higher high-order aggregates, and a mixture thereof. The aggregates of the protein of interest are formed, for example, by the aggregation of a plurality of monomers of the protein of interest.

The antibody protein is a glycoprotein molecule (also referred to as a gamma globulin or an immunoglobulin) that is produced by B lymphocytes in the infection protection mechanism of vertebrates as generally defined in biochemistry. For example, the antibody protein purified with the cation-exchange chromatographic support media according to the embodiment is used as a drug for humans and has substantially the same structure as that of the *in vivo* antibody proteins of humans as recipients.

The antibody protein may be a human antibody protein or may be an antibody protein derived from a non-human mammal (e.g. cattle and a mouse). Alternatively, the antibody protein may be a chimeric antibody protein with human IgG or a humanized antibody protein. The chimeric antibody protein with human IgG is an antibody protein having variable regions derived from a non-human organism such as a mouse and additionally having constant regions replaced with those of a human-derived immunoglobulin. The humanized antibody protein is an antibody protein having a non-human organism-derived complementarity-determining regions (CDRs) in variable regions and additionally having human-derived framework regions (FRs). The humanized antibody protein has much lower immunogenicity than that of the chimeric antibody protein.

The antibody protein, which is one example of a subject to be purified by the purification method according to the embodiment, is not particularly limited by its class (isotype) and subclass. Antibody proteins are classified into, for example, five types of classes, IgG, IgA, IgM, IgD, and IgE, according to difference in constant region structure. However, the antibody protein to be purified with purification method according to the embodiment may be any of these five types of classes. Human antibody proteins have four IgG subclasses, IgG1 to IgG4, and two IgA subclasses, IgA1 and IgA2. However, the antibody protein to be purified with the purification method according to the embodiment may be any of the subclasses. Antibody-related proteins such as Fc fusion proteins composed of a Fc region bound with a protein can also be included in the antibody protein to be purified with the purification method according to the embodiment.

Antibody proteins can also be classified depending on their origins. However, the antibody protein to be purified with the purification method according to the embodiment may be any of natural human antibody proteins, recombinant human antibody proteins produced by a gene recombination technique, monoclonal antibody proteins, and polyclonal antibody proteins. Among these antibody proteins, human IgG is preferred from the viewpoint of a demand and importance as an antibody drug, though the antibody protein is not limited thereto.

The cation-exchange chromatographic support media according to the embodiment comprises a membrane matrix and a copolymer immobilized on the surface of the membrane matrix, wherein the copolymer comprises a (meth)acrylamide-based compound and/or a (meth)acrylate-based compound as monomer units, and the support media has one or more types of cation-exchange groups including at least a weak cation-exchange group at a density higher than 30 mmol/L, preferably a density higher than 40 mmol/L, more preferably a density higher than 45 mmol/L, further preferably a density higher than 100 mmol/L, per volume of the support media.

The "(meth)acrylamide-based compound" is a monomer or a monomer unit having acrylamide as a backbone and may be hydrophilic or hydrophobic depending on a structure other than the backbone. The "(meth)acrylate-based compound" is a monomer or a monomer unit having acrylate as a backbone and may be hydrophilic or hydrophobic depending on a structure other than the backbone.

The "density" means the concentration of cation-exchange groups in the cation-exchange chromatographic support media, and this concentration is generally indicated as the concentration in terms of the number of moles of cation-exchange groups per liter of the cation-exchange chromatographic support media. The "cation" is also referred to as a positive ion, and the "anion" is also referred to as a negative ion. The "support media" is also referred to as an exchanger, an adsorbent, or a stationary phase.

The membrane matrix comprised in the cation-exchange chromatographic support media according to the embodiment is not particularly limited by its shape. Examples of the shape include hollow fiber, flat membranes, nonwoven cloth, monoliths, capillaries, sintered compacts, discs, and cylinders. The material is not particularly limited and is preferably constituted by a polyolefin-based polymer, a polyamide (nylon), a polyester, polyethersulfone, cellulose, or the like.

Examples of the polyolefin-based polymer include: olefin homopolymers such as ethylene, propylene, butylene, and vinylidene fluoride; copolymers of two or more of the olefins; and copolymers of one or two or more of the olefins and a perhalogenated olefin. Examples of the perhalogenated olefin include tetrafluoroethylene and/or chlorotrifluoroethylene. Among them, polyethylene or polyvinylidene fluoride is preferred from the viewpoint of having excellent mechanical strength and producing high adsorption capacity of foreign matter such as proteins. Examples of the polyamide include, but are not particularly limited to, nylon 6 ($\varepsilon$-caprolactam polycondensate), nylon 11 (undecanelactam polycondensate), nylon 12 (lauryllactam polycondensate), nylon 66 (copolycondensate of hexamethylenediamine and adipic acid), nylon 610 (copolycondensate of hexamethylenediamine and adipic acid), nylon 6T (copolycondensate of hexamethylenediamine and terephthalic acid), nylon 9T (copolycondensate of nonanediamine and terephthalic acid), nylon M5T (copolycondensate of methylpentanediamine and terephthalic acid), nylon 621 (copolycondensate of caprolactam and lauryllactam), copolycondensates of p-phenylenediamine and terephthalic acid, and copolycondensates of m-phenylenediamine and isophthalic acid. Examples of the polyester include, but are not particularly limited to, polyethylene terephthalate, polytrimethylene terephthalate, polybutylene terephthalate, polyethylene naphthalate, and polybutylene naphthalate.

The membrane matrix has, for example, a plurality of pores. The pore size is not particularly limited and is, for example, 5 nm or larger and 1000 nm or smaller, preferably 10 nm or larger, more preferably 50 nm or larger, further preferably 100 nm or larger, particularly preferably 150 nm or larger or 400 nm or larger, for a substrate in a hollow fiber form. Also, the pore size is not particularly limited and is preferably 900 nm or smaller, more preferably 800 nm or smaller, further preferably 700 nm or smaller, particularly preferably 650 nm or smaller from the viewpoint of a substrate membrane surface area. A membrane matrix having a pore size of 5 nm or smaller tends to be able to separate antibody proteins having a smaller molecular weight. Also, a membrane matrix having a pore size of 1000 nm or larger has a smaller surface area and tends to have a smaller binding capacity of impurities.

When a graft ratio mentioned later is high or when the percentage of hydrophilic monomers in the copolymer is high, the pores of a hollow fiber membrane tend to be clogged. In such a case, a pore size on the order of 400 nm or larger and 650 nm or smaller is preferred.

When the substrate is in a flat membrane form or in a nonwoven cloth form, the preferred range of the pore size increases because it is possible to use a backing material or it is possible to laminate such substrates for using the substrate as a membrane. The preferred range is 10 nm or larger and 1 mm or smaller, preferably 100 nm or larger, more preferably 200 nm or larger, further preferably 300 nm or larger. Also, the pore size is preferably 500 um or smaller, more preferably 300 um or smaller, further preferably 100 um or smaller, from the viewpoint of a surface area.

The cation-exchange chromatographic support media according to the embodiment has at least a weak cation-exchange group and has one or more types of cation-exchange groups at a density higher than 30 mmol/L per volume of the support media.

Examples of the weak cation-exchange group include a carboxylic acid group, a phosphonic acid group, and a phosphoric acid group. The weak cation-exchange group can vary in charge quantity depending on the pH of a mobile phase. Therefore, the charge density of the cation-exchange chromatographic support media can be adjusted by changing the pH of a mobile phase.

The method for introducing the weak cation-exchange group includes a method of copolymerizing monomers having the weak cation-exchange group. Examples of such monomers include acrylic acid, methacrylic acid, acrylic acid compounds, and methacrylic acid compounds. Examples of the acrylic acid compounds include 2-acryloxyethylsuccinic acid, 2-acryloxyethylhexahydrophthalic acid, and 2-acryloxyethylphthalic acid. Examples of the methacrylic acid compounds include 2-methacryloxyethylsuccinic acid, 2-methacryloxyethylhexahydrophthalic acid, and 2-methacryloxyethylphthalic acid.

The weak cation-exchange group may be introduced by copolymerizing monomers having a group convertible to the weak cation-exchange group, followed by functional group conversion. Such a functional group is an ester or the like. Examples of the monomers include monomers such as t-butyl acrylate, t-butyl methacrylate, benzyl acrylate, benzyl methacrylate, allyl acrylate, and allyl methacrylate. These monomers are used in the copolymerization, and then, the group can be deprotected under acidic conditions and converted to a carboxylic acid group.

The cation-exchange chromatographic support media according to the embodiment may have a strong cation-exchange group as long as having at least one type of weak cation-exchange group, or may only have the weak cation-exchange group without having a strong cation-exchange group. The total density of cation-exchange groups can be higher than 30 mmol/L per volume of the support media.

The introduction of the strong cation-exchange group to the cation-exchange chromatographic support media can render change in charge quantity insensitive to pH and improve reproducibility. Almost all of strong cation-exchange groups have a charge in a pH region of a practical antibody solution in antibody purification and therefore have a constant charge quantity. Thus, the presence of the strong cation-exchange group in the cation-exchange chromatographic support media secures a constant charge quantity equal to or larger than a given level. The charge quantity of the weak cation-exchange group can be adjusted by changing pH so that the charge quantity of the whole cation-exchange chromatographic support media can be finely adjusted. The presence of the strong cation-exchange group in the cation-exchange chromatographic support media can prevent the performance of the cation-exchange chromatographic support media from depending largely on small change in pH. Examples of the strong cation-exchange group include a sulfonic acid group.

A total density of cation-exchange groups lower than 30 mmol/L tends to decrease the amount of cation-exchange groups that may be charged, reduce adsorption capacity, and lower the amount of antibodies that can be processed. In Patent Literature 3, the selectivity of flow-through purification between antibody monomers and aggregates is exerted by using strong cation-exchange groups and setting the density of cation-exchange groups to 30 mmol/L or lower. However, such a low density of cation-exchange groups decreases adsorption capacity and lowers the amount of antibodies that can be processed. A density of cation-exchange groups equal to or lower than 30 mmol/L tends to narrow a possible charge quantity range and narrow the types of applicable antibodies.

The total density of cation-exchange groups per volume of the support media can be higher than 30 mmol/L. When the density of weak cation-exchange groups among these cation-exchange groups is 5 mol/L or higher, preferably 10 mol/L or higher, more preferably 15 mmol/L or higher, the charge density can be adjusted. Also, the support media may have one or more types of cation-exchange groups including at least a weak cation-exchange group at a density lower than 1000 mmol/L, preferably a density lower than 700 mmol/L, more preferably a density lower than 500 mmol/L, further preferably a density lower than 400 mmol/L, per volume of the support media. The total density of cation-exchange groups within this range permits efficient separation of the aggregates of the protein of interest.

The copolymer comprised in the cation-exchange chromatographic support media according to the embodiment is immobilized on the membrane matrix and immobilized on the membrane matrix, for example, through a covalent bond. This support media has at least a weak cation-exchange group and can finally have one or more types of cation-exchange groups at a density higher than 30 mmol/L.

The method for immobilizing the copolymer onto the membrane matrix includes graft polymerization. Examples of the graft polymerization method include radiation graft polymerization and surface living radical polymerization methods.

In the case of immobilizing the copolymer onto the surface of the membrane matrix by the radiation graft polymerization method, any approach may be adopted for generating radicals on the membrane matrix. The irradiation of the membrane matrix with ionizing radiation generates homogeneous radicals throughout the membrane matrix and is therefore preferred. For example, $\gamma$ ray, electron ray, $\beta$ ray, and neutron ray can be used as the types of the ionizing radiation. Electron ray or $\gamma$ ray is preferred for industrial-scale execution. The ionizing radiation is obtained from a radioisotope such as cobalt 60, strontium 90, or cesium 137 or using an X-ray photography apparatus, an electron beam accelerator, an ultraviolet irradiation apparatus, or the like.

The irradiation dose of the ionizing radiation is preferably 1 kGy or larger and 1000 kGy or smaller, more preferably 2 kGy or larger and 500 kGy or smaller, further preferably 5 kGy or larger and 200 kGy or smaller. An irradiation dose of smaller than 1 kGy tends to be less likely to generate homogeneous radicals. Also, an irradiation dose exceeding 1000 kGy tends to cause reduction in the physical strength of the membrane matrix.

In general, graft polymerization methods based on irradiation with ionizing radiation are broadly classified into: a preirradiation method which involves generating radicals on the membrane matrix and subsequently contacting the radicals with reactive compounds; and a simultaneous irradiation method which involves generating radicals on the membrane matrix with the membrane matrix contacted with reactive compounds. Any method may be applied to this embodiment, and the preirradiation method is preferred which rarely forms oligomers.

The solvent for use in the polymerization for the copolymer according to the embodiment is not particularly limited as long as the solvent can uniformly dissolve the reactive compounds. Examples of such a solvent include: alcohols such as methanol, ethanol, isopropanol, and t-butyl alcohol; ethers such as diethyl ether and tetrahydrofuran; ketones such as acetone and 2-butanone; water; and mixtures thereof.

The copolymer comprises, in its composition, one or more types of monomer units selected from compounds of acrylamides, methacrylamides, acrylates, and methacrylates in addition to monomer units having the cation-exchange groups. The copolymer may consist only of any combination of acrylamides, methacrylamides, acrylates, and methacrylates or may consist only of methacrylates. The copolymer may further comprise, in its composition, one or more types of hydrophilic and/or hydrophobic compounds, which are neutral monomers having no charge, as monomer units. These monomer units tend to be stable against general acidic or basic conditions for washing the cation-exchange chromatography, be less likely to undergo reduction in performance caused by washing, and be less likely to reduce membrane strength. It is considered that these hydrophilic and/or hydrophobic monomers are copolymerized with the monomers having the cation-exchange groups so that the distance between the cation-exchange groups is widened and more selective adsorption of antibody aggregates is facilitated. Examples of an additional monomer unit include acrylonitrile. However, acrylonitrile is easily hydrolyzed by an alkali. Therefore, acrylonitrile, when used as a monomer unit, is not preferred because its performance tends to be impaired by alkali washing. Monomer units containing an aromatic group, such as styrenes, are not preferred because these monomer units tend to render the membrane stiff and fragile. The mass percentage of the hydrophilic monomer unit and/or the hydrophobic monomer unit as neutral monomers in the copolymer is preferably higher than, more preferably twice or more, further preferably 3 or more times that of the monomer units having the cation-exchange from the viewpoint of differentiating adsorption power for antibody monomers from adsorption power for antibody aggregates.

In the embodiment, the copolymer consists only of a cation-exchange group-containing monomer and a neutral monomer. Therefore, the mass of the cation-exchange group-containing monomer and the mass of the neutral monomer can be determined as follows:

(Mass of the cation-exchange group-containing monomer) =

(Cation-exchange group density × Support media volume × Molecular weight of the cation-exchange group-containing monomer)

(Mass of the neutral monomer) =

(Mass of the cation-exchange support media — Mass of the substrate support media — Mass of the cation-exchange group-containing monomer)

The mass percentage of the cation-exchange group-containing monomer unit and the mass percentage of the neutral monomer unit can be determined from these mass ratios.

Since antibodies have properties based on hydrophobic interaction, the antibodies may be adsorbed onto a membrane matrix through hydrophobic interaction resulting from the strong hydrophobicity of the membrane matrix. As a result, the recovery rate of an antibody of interest may be reduced. In order to cope with this phenomenon, the adsorption of antibodies onto the membrane matrix can be prevented by introducing a hydrophilic monomer during the polymerization for the copolymer. Examples of such a hydrophilic monomer include acrylamide, methacrylamide, and (meth)acrylamide compounds such as dimethylacrylamide, dimethylmethacrylamide, diethylacrylamide, diethylmethacrylamide, N-methylacrylamide, N-methylmethacrylamide, N-ethylacrylamide, N-ethylmethacrylamide, N-isopropylacrylamide, N-isopropylmethacrylamide, N-(hydroxymethyl)acrylamide, N-(hydroxymethyl)methacrylamide, N-(2-hydroxyethyl)acrylamide, and N-(2-hydroxyethyl)methacrylamide. Alternatively, examples of the hydrophilic monomer as described above include acrylate, methacrylate, and (meth)acrylate compounds such as 2-hydroxyethyl acrylate, 2-hydroxyethyl methacrylate, 3-hydroxypropyl acrylate, 3-hydroxypropyl methacrylate, 4-hydroxybutyl acrylate, 4-hydroxybutyl methacrylate, 2-(dimethylamino)ethyl acrylate, and 2-(dimethylamino)ethyl methacrylate.

Alternatively, the adsorption selectivity for antibody aggregates over antibody monomers may be enhanced by introducing a hydrophobic monomer during the polymerization for the copolymer and thereby exploiting the hydrophobic interaction. In general, antibody aggregates exhibit stronger hydrophobic interaction than that of antibody monomers. The selection of a suitable hydrophobic monomer can achieve the marked difference in hydrophobic interaction between antibody monomers and antibody aggregates and high selectivity. Examples of such a hydrophobic monomer include styrenes, alkylacrylamides, alkylmethacrylamides, alkyl acrylates, and alkyl methacrylates. Alkylacrylamides, alkylmethacrylamides, alkyl acrylates, and alkyl methacrylates are desirable from the viewpoint of dynamic strength. The alkyl group can be any linear or branched alkyl group having four or more carbon atoms, and the resulting monomer can substantially cause hydrophobic interaction with antibodies.

The copolymer may neither comprise a monomer unit containing two or more polymerizable functional groups nor may have a cross-linked structure. Alternatively, the copolymer may comprise a monomer unit containing two or more polymerizable functional groups and may have a cross-linked structure. However, preferably, the copolymer substantially has a non-cross-linked structure. The substantially non-cross-linked copolymer refers to a copolymer having a low degree of cross-linking that does not substantially influence antibody aggregate adsorption performance even if having a cross-linked structure. When the copolymer is substantially non-cross-linked, the mass percentage of the monomer unit containing two or more polymerizable functional groups is, for example, 10% or lower, 5% or lower, 4% or lower, 3% or lower, 2% or lower, 1% or lower, 0.5% or lower, or 0.1% or lower. A lower mass percentage thereof is more preferred.

Examples of the monomer containing two or more polymerizable functional groups include, but are not particularly limited to, monomers having olefins as the polymerizable functional groups. Examples of such a monomer include (meth)acrylamide-based monomers, (meth)acrylate-based monomers, and monomers having a mixture of their functional groups. Examples of the (meth)acrylamide-based monomers include N,N'-methylenebisacrylamide, N,N'-ethylenebisacrylamide, N,N'-propylenebisacrylamide, N,N'-(1,2-dihydroxyethylene)bisacrylamide, N,N'-methylenebismethacrylamide, N,N'-ethylenebismethacrylamide, N,N'-propylenebismethacrylamide, and N,N'-(1,2-dihydroxyethylene)bismethacrylamide.

Examples of the (meth)acrylate-based monomers include ethylene glycol diacrylate, 1,3-butanediol diacrylate, 1,4-butanediol diacrylate, 1,5-pentanediol diacrylate, 1,6-hexanediol diacrylate, 1,9-nonanediol diacrylate, 1,10-decanediol diacrylate, neopentyl glycol diacrylate, diethylene glycol diacrylate, triethylene glycol diacrylate, polyethylene glycol diacrylate, dipropylene glycol diacrylate, tripropylene glycol diacrylate, 2-hydroxy-1,3-propanediol diacrylate, 4,4'-thiodibenzenethiol diacrylate, trimethylolpropane triacrylate, pentaerythritol tetraacrylate, ethylene glycol dimethacrylate, 1,3-butanediol dimethacrylate, 1,4-butanediol dimethacrylate, 1,5-pentanediol dimethacrylate, 1,6-hexanediol dimethacrylate, 1,9-nonanediol dimethacrylate, 1,10-decanediol dimethacrylate, neopentyl glycol dimethacrylate, diethylene glycol dimethacrylate, triethylene glycol dimethacrylate, polyethylene glycol dimethacrylate, dipropylene glycol dimethacrylate, tripropylene glycol dimethacrylate, 2-hydroxy-1,3-propanediol dimethacrylate, 4,4'-thiodibenzenethiol dimethacrylate, trimethylolpropane trimethacrylate, and pentaerythritol tetramethacrylate.

The optimum value of the graft chain binding ratio (graft ratio) of the graft polymerization may differ depending on the density of the substrate membrane. When the substrate membrane is polyethylene, the graft ratio is preferably 20% or more, more preferably 25% or more, further preferably 30% or more, from the viewpoint of adsorption capacity. Also, the graft ratio is preferably 200% or less, more preferably 150% or less, further preferably 100% or less, from the viewpoint of securing dynamically stable strength. The graft ratio is represented by the following expression:

$$dg~(\%)~=~(w_1~-~w_0)~/~w_0~\times~100$$

wherein $w_0$ represents the weight of the porous hollow fiber before reaction, and $w_1$ represents the weight of the graft chain-introduced porous hollow fiber.

When the substrate membrane is polyvinylidene fluoride, the suitable graft ratio differs from that of polyethylene because polyvinylidene fluoride has a higher density than that of polyethylene. When the substrate membrane is polyvinylidene fluoride, the graft ratio is preferably 5% or more, more preferably 10% or more, further preferably 15% or more, from the viewpoint of adsorption capacity. Also, the graft ratio is preferably 100% or less, more preferably 80% or less, further preferably 70% or less, from the viewpoint of securing dynamically stable strength.

The cation-exchange group-containing copolymer immobilized on the membrane matrix permits steric adsorption as compared with cation-exchange groups distributed on the surface of the membrane matrix. Therefore, antibody aggregates are more strongly adsorbed on the substrate than antibody monomers so that the antibody monomers can be obtained with high purity.

Thus, the copolymer preferably has at least a weak cation-exchange group and has one or more types of cation-exchange groups. A copolymer having a cross-linked structure interferes with the steric adsorption of antibody aggregates onto the substrate, though this copolymer has the advantage that the pressure of a passing liquid is reduced by suppressing the rising of the copolymer. Therefore, it is desirable that the copolymer should substantially have a non-cross-linked structure, from the viewpoint of more strongly adsorbing antibody aggregates than antibody monomers so that the antibody monomers can be obtained with high purity.

If the copolymer contains a small amount of hydrophilic monomers, this copolymer is elongated or contracted and makes it impossible to sterically adsorb antibody aggregates. Therefore, the steric adsorption of antibody aggregates requires a content thereof above a certain level. The mass percentage of the hydrophilic monomers in such a copolymer is preferably 30% or more, more preferably 40% or more, further preferably 50% or more, particularly preferably 60% or more. It is desirable that the mass percentage of the hydrophilic monomers should be higher than that of hydrophobic monomers. The mass percentage of the hydrophilic monomers is preferably 1.3 or more times, more preferably 1.5 or more times, further preferably 1.7 or more times, particularly preferably 1.9 or more times that of the hydrophobic monomers. The mass percentages of the hydrophilic monomers and the hydrophobic monomers can be detected from the final product by pyrolysis GC/MS. Alternatively, these mass percentages can also be calculated, assuming that the mass percentages agree with mixing composition ratios for synthesis. For the graft polymerization, the mass ratio of the hydrophilic monomers in the copolymer corresponds to the weight ratio of the hydrophilic monomers to all monomers mixed.

The method for determining the total cation-exchange group density also, which is the total of the strong cation-exchange group density and the weak cation-exchange group density, includes a method which involves protonating all of the cation-exchange groups using a strong acid or the like, followed by neutralization using a base and measurement by back titration. Another method involves replacing counter cations of the cation-exchange groups with lithium ions, followed by treatment with a strong acid and the measurement of the amount of lithium eluted. The total cation-exchange group density can be measured by these methods.

The method for measuring the strong cation-exchange group density can involve, for example, protonating all of the strong cation-exchange groups using a strong acid or the like, followed by the addition of an aqueous sodium chloride solution and the titration of eluted hydrogen chloride for measurement.

The weak cation-exchange group density can be determined by subtracting the strong cation-exchange group density from the total cation-exchange group density.

The purification method according to the embodiment is a purification method for purifying a protein monomer from a mixed solution containing the protein monomer and protein aggregates, the purification method comprising contacting the mixed solution with the aforementioned cation-exchange chromatographic support media. The recovery rate of the protein monomer by the aforementioned cation-exchange chromatographic support media is, for example, 78% or more, 80% or more, 85% or more, 90% or more, 91% or more, 92% or more, 93% or more, 94% or more, 95% or more, 96% or more, 97% or more, 98% or more, or 99% or more. The recovery rate of the monomer of the protein of interest is calculated according to the expression given below. In the expression given below, the amount of an aqueous solution processed, containing the protein of interest is the total amount of an aqueous solution containing the protein of interest, introduced to the cation-exchange chromatographic support media.

$$
\begin{aligned}
\text{Recovery rate (\%) of the protein of interest} = \\
(\text{Amount (mL) of an aqueous solution of the protein of} \\
\text{interest recovered} \times \text{Content (mg/mL) of the monomer of} \\
\text{the protein of interest in a recovered aqueous solution}) \\
/ (\text{Amount (mL) of an aqueous solution processed,} \\
\text{containing the protein of interest} \times \text{Content(mg/mL) of} \\
\text{the monomer of the protein of interest in an aqueous} \\
\text{solution before the processing}) \times 100
\end{aligned}
$$

The flow-through (purification) refers to a purification method aimed at allowing a monomer of the protein of interest to flow through the cation-exchange chromatographic support media. For example, when a monomer of an antibody protein is matter of interest and aggregates of an antibody protein are impurities, the antibody protein monomer generally flows on the surface of the cation-exchange chromatographic support media whereas the antibody protein aggregates are generally adsorbed onto the cation-exchange chromatographic support media. In this respect, the antibody protein monomer may be adsorbed onto the cation-exchange chromatographic support media, but is purified by the more selective adsorption of the antibody protein aggregates onto the adsorbent.

The mobile phase to be introduced to the cation-exchange chromatographic support media can employ a buffer solution (buffer) other than a strong acid and a strong alkali, and some mobile phases do not require an organic solvent. In this context, the buffer solution is an aqueous solution containing a salt. Specific examples thereof include phosphate buffer solutions, tris buffer solutions, and acetate buffer solutions. The buffer solution is not particularly limited as long as the buffer solution is usually used. The concentration of the salt is 0 mmol/L or higher and 100 mmol/L or lower, preferably 0 mmol/L or higher and 50 mmol/L or lower, more preferably 0 mmol/L or higher and 30 mmol/L or lower. The buffer concentration is 1 mmol/L or higher and 100 mmol/L or lower, preferably 2 mmol/L or higher and 70 mmol/L or lower, further preferably 5 mmol/L or higher and 50 mmol/L or lower. In this context, the buffer concentration refers to the concentration of an active ingredient in the buffer. For example, the acetate buffer is usually prepared from acetic acid and sodium acetate, and the buffer concentration of the acetate buffer is the total concentration of acetic acid and sodium acetate. The buffer concentration of the tris buffer refers to the concentration of trishydroxymethylaminomethane. The buffer concentration of a buffer indicated by an acetate-tris buffer or the like is the concentration of the former component. The buffer concentration of acetate-tris is the concentration of acetate while the buffer concentration of tris-acetate is the concentration of tris. In the present embodiment, a solution of pH hardly having buffering ability (e.g., an acetate buffer solution of pH 3.4) can also be used in the chromatography.

A concentration of an inorganic salt higher than 100 mmol/L in the buffer solution tends to reduce the degree of dissociation of ion-exchange groups and make it difficult for the cation-exchange chromatographic support media to carry impurities such as aggregates.

The electric conductivity of an aqueous solution containing the protein of interest is 0.5 mS/cm or higher and 20 mS/cm or lower, more preferably 0.5 mS/cm or higher and 15 mS/cm or lower, further preferably 0.5 mS/cm or higher and 10 mS/cm or lower, particularly preferably 0.8 mS/cm or higher and 5.0 mS/cm or lower. An electric conductivity exceeding 20 mS/cm tends to increase a masking effect and make it difficult for the cation-exchange chromatographic support media to carry impurities such as aggregates.

The hydrogen ion concentration of the buffer solution differs depending on the isoelectric point, size, etc., of antibody proteins to be processed, and the optimum conditions need to be appropriately studied. The pH is, for example, pH 4.0 or higher and pH 10.0 or lower, preferably pH 5.0 or higher and pH 9.5 or lower, further preferably pH 6.0 or higher and pH 9.0 or lower, particularly preferably pH 6.5 or higher and pH 8.0 or lower. pH near the isoelectric point of antibody proteins tends to decrease the electrostatic repulsion between antibody proteins, which are easily aggregated. Also, pH of lower than 4.0 tends to denature antibody proteins and cause reduction in activity or reduction in quality such as the purification of aggregates.

The amount of antibodies loaded in the cation-exchange chromatography step is not particularly limited as long as impurities can be removed. The amount of antibodies loaded is 100 mg or larger per mL of the support media and is preferably 300 mg or larger, more preferably 500 mg or larger, further preferably 700 mg or larger, still further preferably 0.8 g or larger, particularly preferably 1 g or larger, per mL of the support media from the viewpoint of efficient purification.

The cation-exchange chromatography step that is carried out in the present embodiment reduces the percentage of antibody aggregates by 50% or more, more preferably 60% or more, further preferably 70% or more, when 100 mg or larger of antibodies including monomers and the aggregates is flow-through purified with respect to 1 mL of the support media.

The purified solution of monomers obtained in the purification step is introduced to a virus removal membrane having a virus logarithmic reduction value of 3 or more. In this context, aggregates may be formed from monomers if the purified solution of monomers is concentrated or the acidity or alkalinity of the purified solution is adjusted. Therefore, it is preferred that no additional step should be comprised between the purification step and the virus removal step. Also, aggregates may be formed from monomers as time advances. Therefore, it is preferred that the purified solution of monomers obtained in the purification step should be immediately introduced to the virus removal membrane without being stored. For example, the purification step and the virus removal step may be performed by a continuous process.

The virus removal membrane is not particularly limited as long as its virus logarithmic reduction value is 3.00 or more. For example, a virus removal membrane disclosed in International Publication No. WO 2003/026779, 2004/035180, 2015/156401, or 2015/156403 may be used.

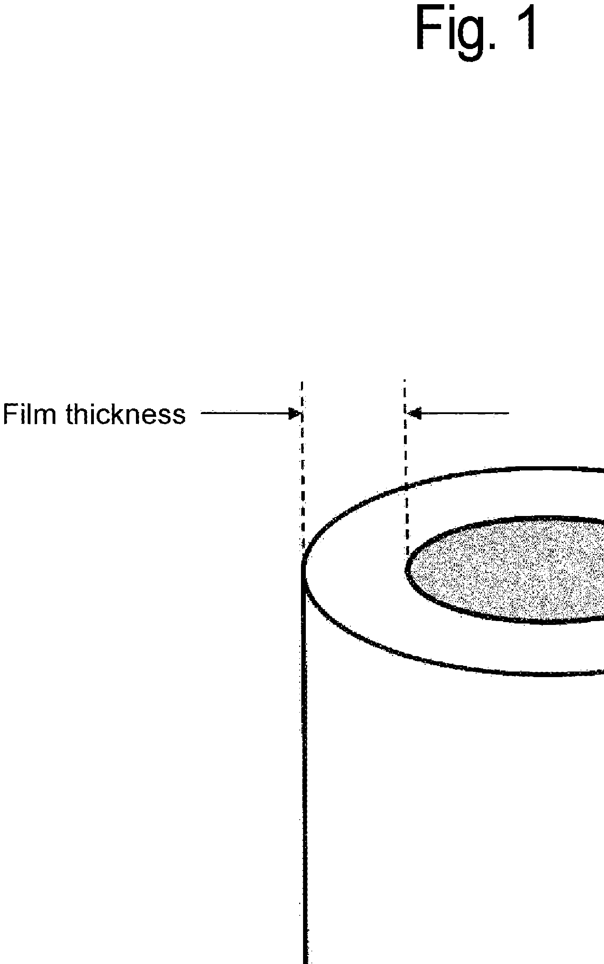

For example, as shown in Figure 1, virus removal membrane 10 has primary-side surface 1 to which a protein-containing solution is to be applied, and secondary-side surface 2 from which a permeate that has penetrated the virus removal membrane 10 is to be flowed.

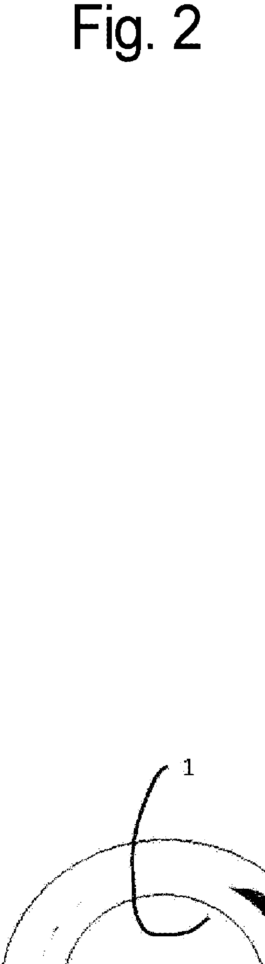

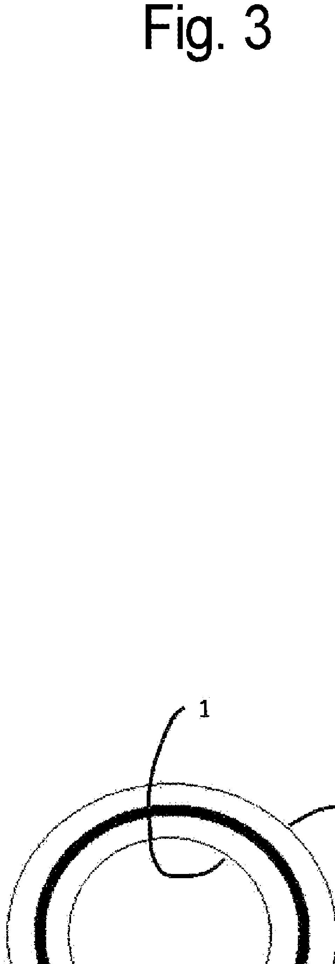

Small viruses to be removed by the virus removal membrane 10 have a diameter of, for example, 10 to 30 nm or 18 to 24 nm. Specific examples of the viruses include parvovirus. The parvovirus has a diameter of approximately 20 nm. The virus removal membrane 10 has a virus capture site which captures viruses, on the cross section thereof. In the virus removal membrane 10, the amount of viruses captured at the virus capture site is preferably uniform on the cross section, irrespective of a location on the filtration surface (primary-side surface 1) to be entered by a solution. This is because, when the amount of viruses captured by the virus removal membrane 10 is non-uniform depending on a location on the filtration surface, a solution is focused on a certain site on the filtration surface so that the amount of viruses loaded on the site is partially increased, leading to the possibility that viruses leak from the site at the time of large-capacity filtration under highpressure conditions. When the virus removal membrane 10 has the shape of a hollow fiber membrane, the amount of viruses captured at the virus capture site is preferably uniform as shown in Figure 3, without being non-uniform as shown in Figure 2, in the circumferential direction.

The thickness of the site capturing viruses in the virus removal membrane 10 is preferably uniform within the virus capture site. When the virus removal membrane 10 has the shape of a hollow fiber membrane, the thickness of the virus capture site is preferably uniform in the circumferential direction. This is because such a uniform thickness of the virus capture site allows a solution to be uniformly spread in the circumferential direction and decreases the possibility that viruses leak.

The structure of the virus removal membrane 10 is preferably an asymmetric structure where the pore size of holes decreases in turn increases from the primary side toward the secondary side. The virus capture site comprises a site having the smallest pore size of holes on the cross section of the virus removal membrane 10. The structure comprising the site having the smallest pore size of holes is effective for improvement in the capability of removing viruses.

In this context, viruses captured by the virus removal membrane 10 may be difficult to visually detect. By contrast, gold colloids, albeit having a diameter equivalent to that of viruses, do not permit light penetration and is therefore easy to visually detect. Therefore, the characteristics of the virus removal membrane 10 can be evaluated, for example, by filtering a solution containing gold colloids through the virus removal membrane 10 and then measuring the relative brightness of a gold colloid capture site of the gold colloid-captured virus removal membrane 10 on the cross section of the virus removal membrane 10.

In the virus removal membrane 10 according to the embodiment, a solution containing gold colloids having a diameter of 20 nm is applied to the virus removal membrane 10 from the primary-side surface 1 to capture the gold colloids onto the virus removal membrane 10. When the brightness on the cross section of the virus removal membrane 10 is measured, a value obtained by dividing the standard deviation of spectrum area values of displacement of the brightness by an average spectrum area of displacement of the brightness is 0.01 or more and 1.50 or less. This value indicates a coefficient of variation of the amount of gold colloids captured by the virus removal membrane 10. A smaller value expresses higher uniformity of the amount of gold colloids captured at the gold colloid capture site in the virus removal membrane 10.

The aforementioned value indicating the coefficient of variation as to the virus removal membrane 10 according to the embodiment is 0.01 or more and 1.50 or less, 0.01 or more and 1.40 or less, 0.01 or more and 1.30 or less, 0.01 or more and 1.20 or less, 0.01 or more and 1.10 or less, or 0.01 or more and 1.00 or less. A coefficient of variation less than 0.01 is the measurement limit. A coefficient of variation larger than 1.50 leads to the possibility that viruses leak because a solution may be focused on at least a certain site in the circumferential direction of the membrane.

The aforementioned coefficient of variation of 0.01 or more and 1.50 or less allows viruses to be uniformly captured at the virus capture site (in the circumferential direction as to a hollow fiber membrane) of the membrane and can keep high virus removal performance even when the total amount of viruses loaded on the virus removal membrane (the amount of viruses spiked to a pharmaceutical protein or the total filtration volume) is increased.

The aforementioned coefficient of variation is measured by, for example, the following method: a section is cut out of the virus removal membrane after filtration of a gold colloid solution, and the brightness profiles of a plurality of sites stained with gold colloids on the cross section of the section are measured under an optical microscope. Since gold colloids absorb light, the displacement of the brightness depends on the amount of gold colloids captured. If necessary, background noise may be removed from the brightness profiles. Then, a graph having a film thickness on the abscissa and the displacement of the brightness on the ordinate is prepared, and spectrum areas of displacement of the brightness appearing on the graph are calculated. Further, a standard deviation of the spectrum areas of displacement of the brightness at the plurality of sites is divided by an average spectrum area of displacement of the brightness at the plurality of sites to calculate a value that indicates the coefficient of variation of the amount of gold colloids captured at the gold colloid capture site in the virus removal membrane 10.

The thickness of the site capturing gold colloids having a diameter of 20 nm or larger and 30 nm or smaller, on the cross section of the virus removal membrane 10 in a wet state is 10.0 $\mu$m or larger and 30.0 $\mu$m or smaller, 10.0 $\mu$m or larger and 25.0 $\mu$m or smaller, 10.0 $\mu$m or larger and 22.0 $\mu$m or smaller, or 10.0 $\mu$m or larger and 20.0 $\mu$m or smaller, preferably 11.0 $\mu$m or larger and 20.0 $\mu$m or smaller, more preferably 12.0 $\mu$m or larger and 20.0 $\mu$m or smaller, further preferably 13.0 $\mu$m or larger and 20.0 $\mu$m or smaller. A gold colloid capture site having a thickness larger than 30.0 $\mu$m tends to reduce filtration efficiency not only for a gold colloid-containing solution but for a virus-containing solution. A gold colloid capture site thinner than 10 $\mu$m is not preferred because there is a possibility that viruses leak when the total amount of viruses loaded on the virus removal membrane (the amount of viruses spiked to a pharmaceutical protein or the total filtration volume) is increased.

The thickness of the site capturing gold colloids having the diameter of 20 nm or larger and 30 nm or smaller is obtained by, for example, the following method: a section is cut out of the virus removal membrane after filtration of a solution of gold colloids having the diameter of 20 nm or 30 nm. The brightness profiles of a plurality of sites stained with gold colloids on the cross section of the section are measured under an optical microscope. Then, first distance "a" from the primary-side surface 1 of the virus removal membrane 10 to a site of the gold colloid capture site where is closest to the primary-side surface is measured in the film thickness direction. Also, second distance "b" from the primary-side surface 1 of the virus removal membrane 10 to a site of the gold colloid capture site where is closest to the secondary-side surface 2 is measured in the film thickness direction.

Next, value "A" indicated by percentage of the first distance "a" divided by film thickness "c" of the wet virus removal membrane (A = percentage of a/c) is calculated for each of the plurality of sites, and an average of the values "A" of the plurality of sites is calculated as a first reach. Also, value "B" indicated by percentage of the second distance "b" divided by the film thickness "c" of the wet virus removal membrane (B = percentage of b/c) is calculated for each of the plurality of sites, and an average of the values "B" of the plurality of sites is calculated as a second reach.

As shown in the expression (1) given below, the difference between average second reach "$B_{20}$" of the virus removal membrane that has filtered the gold colloids having a diameter of 20 nm and average first reach "$A_{30}$" of the virus removal membrane that has filtered gold colloids having a diameter of 30 nm is multiplied by average $C_{AVE}$ of

average film thickness "$C_{20}$" of the wet virus removal membrane that has filtered the gold colloids having the diameter of 20 nm and average film thickness "$C_{30}$" of the wet virus removal membrane that has filtered the gold colloids having the diameter of 30 nm to calculate thickness "T" of the site capturing gold colloids having the diameter of 20 nm or larger and 30 nm or smaller on the cross section of the virus removal membrane 10 when the gold colloids having the diameter of 20 nm and the gold colloids having the diameter of 30 nm are circulated. The thickness "T" of the gold colloid capture site is also referred to as thickness "T" of a closely packed layer of the virus removal membrane.

$$T = (B_{20} - A_{30}) \times C_{AVE} \quad (1)$$

In the aforementioned method, the site capturing gold colloids having the diameter of 20 nm or larger and 30 nm or smaller is determined as the thickness of a region between the first reached position in the virus removal membrane that has filtered the gold colloids having the diameter of 30 nm and the second reached position in the virus removal membrane that has filtered the gold colloids having the diameter of 20 nm. Gold colloids having any diameter of 20 nm or larger and 30 nm or smaller has been confirmed to be captured on the aforementioned range except for errors.

In the case of filtering a solution containing gold colloids having a diameter of 30 nm through the virus removal membrane 10, a site capturing the gold colloids having the diameter of 30 nm on the cross section of the virus removal membrane 10 in a wet state is located at, for example, 15% or more and 60% or less or 20% or more and 55% or less of the film thickness from the primary-side surface 1 when measured under an optical microscope. A membrane that captures the gold colloids having the diameter of 30 nm by a site less than 15% of the film thickness from the primary-side surface is more likely to be clogged because viruses or impurities are captured at a position close to the primary-side surface of the membrane. A membrane that captures the gold colloids having the diameter of 30 nm by a site more distant than 60% of the film thickness from the primary-side surface might be incapable of capturing viruses because viruses of interest are captured at a position close to the secondary-side surface of the membrane. Even if a small amount of gold colloids having a diameter of 30 nm is captured onto a region less than 15% or more distant than 60% of the film thickness from the primary-side surface 1, the capture of the gold colloids onto the region can be regarded as being within the margin of errors from the viewpoint of the ability of the virus removal membrane to remove viruses, when the absolute value of a spectrum as to displacement of brightness determined by subtracting a measured brightness profile from a constant (255) in observation under an optical microscope is 10% or less of the largest absolute value of the spectrum. Thus, in this case, the site capturing the gold colloids having the diameter of 30 nm can be regarded as being located at 15% or more and 60% or less of the film thickness from the primary-side surface 1.

Depending on a membrane structure, the site capturing gold colloids may be formed continuously or intermittently in the thickness direction by passing the gold colloids in the film thickness direction from the primary-side surface to the secondary-side surface. For the virus removal membrane according to the embodiment, it is preferred that the site capturing gold colloids should be continuously formed from the inner side of the primary-side surface to the inner side of the secondary-side surface. The gold colloid capture site continuously formed without interruption in the direction of a passing liquid hinders clogging.

In the case of filtering a solution containing gold colloids having a diameter of 20 nm through the virus removal membrane 10, a site capturing the gold colloids having the diameter of 20 nm on the cross section of the virus removal membrane 10 in a wet state is located at, for example, 25% or more and 85% or less or 30% or more and 80% or less of the film thickness from the primary-side surface 1 when measured under an optical microscope. A membrane that captures gold colloids having a diameter of 20 nm by a site less than 25% of the film thickness from the primary-side surface is more likely to be clogged because viruses or impurities are captured at a position close to the primary-side surface of the membrane. A membrane that captures gold colloids having a diameter of 20 nm by a site more distant than 85% of the film thickness from the primary-side surface might be incapable of capturing viruses because viruses of interest are captured at a position close to the secondary-side surface of the membrane. As in the case of gold colloids having a diameter of 30 nm, even if gold colloids are observed in a region less than 25% or more distant than 85% of the film thickness from the primary-side surface 1, this can be regarded as being within the margin of errors from the viewpoint of the ability of the virus removal membrane to remove viruses, when the absolute value of a spectrum as to displacement of brightness determined by subtracting a measured brightness profile from a constant (255) in observation under an optical microscope is 10% or less of the largest absolute value of the spectrum. For the virus removal membrane according to the embodiment, it is preferred that the site capturing gold colloids having a diameter of 20 nm should be continuously formed in the film thickness direction from the inner side of the primary-side surface to the inner side of the secondary-side surface.

In the case of filtering a solution containing gold colloids having a diameter of 15 nm through the virus removal membrane 10, a site capturing the gold colloids having the diameter of 15 nm on the cross section of the virus removal membrane 10 in a wet state is located at, for example, 60% or more and 90% or less, preferably 60% or more and 89% or less, 60% or more and 88% or less, or 60% or more and 87% or less, of the film thickness from the primary-side surface 1 when measured under an optical microscope. Particularly, the site located at 87% or less of the film thickness is preferred for the capture of viruses. 86% or less of the film thickness is more preferred. A membrane that captures gold colloids having a diameter of 15 nm by a site less than 60% of the film thickness from the primary-side surface is more likely to be clogged because viruses or impurities are captured at a position close to the primary-side surface of the membrane. A membrane that captures gold colloids having a diameter of 15 nm by a site more distant than 90% of the film thickness from the primary-side surface might be incapable of capturing viruses because viruses of interest are captured at a position close to the secondary-side surface of the membrane. As in the case of gold colloids having a diameter of 30 nm or 20 nm, even if gold colloids are observed in a region less than 60% or more distant than 90% of the film thickness from the primary-side surface 1, this can be regarded as being within the margin of errors from the viewpoint of the ability of the virus removal membrane to remove viruses, when the absolute value of a spectrum as to displacement of brightness determined by subtracting a measured brightness profile from a constant (255) in observation under an optical microscope is 10% or less of the largest absolute value of the spectrum. For the virus removal membrane according to the embodiment, it is preferred that a layer of the site capturing gold colloids having a diameter of 15 nm should be continuously formed in the film thickness direction from the inner side of the primary-side surface to the inner side of the secondary-side surface.

The capture position of gold colloids having a diameter of 30 nm, 20 nm or 15 nm is measured only for gold colloids captured by the membrane. Thus, gold colloids that have penetrated the membrane without being captured by the membrane is excluded from the measurement. In short, the capture position is not measured for all gold colloids filtered through the membrane, but is measured as the capture position on the membrane of gold colloids captured by the membrane.

In the case of filtering a solution containing gold colloids having a diameter of 10 nm through the virus removal membrane 10, the gold colloids having a diameter of 10 nm are rarely captured on the cross section of the virus removal membrane 10. This can be confirmed by observation using an optical microscope (Biozero, BZ8100, manufactured by Keyence Corp.) such that a brightness spectrum cannot be detected as a significant value. This can also be confirmed by a decreased logarithmic reduction value. The absence of capture of gold colloids having a diameter of 10 nm means that high permeability can be achieved for useful proteins, such as IgG, having a diameter on the order of 10 nm.

The material of the virus removal membrane 10 consists of cellulose. Regenerated cellulose, natural cellulose, cellulose acetate, or the like can be used as the cellulose. Examples of a method for producing the regenerated cellulose include a method of preparing the regenerated cellulose from a copper ammonia cellulose solution (copper ammonia method) and a method of preparing the regenerated cellulose by the saponification of cellulose acetate with an alkali (saponification method).

Alternatively, the material of the virus removal membrane 10 may be a synthetic polymer such as a thermoplastic crystalline resin. Polyolefin resin or fluorine-based resin is preferred from the viewpoint of the balance between heat resistance and moldability. A hydrophobic thermoplastic crystalline resin may cause the adsorption of proteins and the like, the contamination of the membrane, and the clogging of the membrane, etc. and is therefore preferably rendered hydrophilic. For example, a hydrophilic graft chain may be attached to a membrane consisting of the hydrophobic thermoplastic crystalline resin by a graft polymerization method.

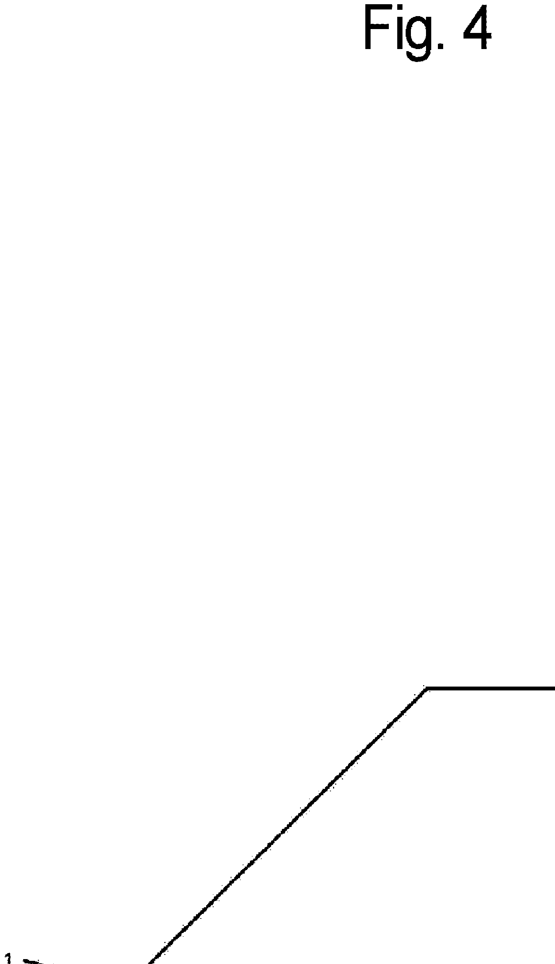

The virus removal membrane 10 has, for example, the shape of a hollow fiber membrane. Alternatively, the virus removal membrane 10 may have the shape of a flat membrane as shown in Figure 4. The hollow fiber membrane, even if having a large membrane area, can be loaded to a container to prepare a small filter.

The film thickness of the virus removal membrane 10 shown in Figure 1 is, for example, 24 μm or larger and 41 μm or smaller, preferably 24 μm or larger and 40 μm or smaller, more preferably 24 μm or larger and 35 μm or smaller, further preferably 24 μm or larger and 30 μm or smaller, in a dry state. A film thickness smaller than 24 μm reduces the strength of the membrane. The resulting membrane might not resist filtration pressure. A film thickness larger than 41 μm might decrease a filtration rate.

The average pore size of holes in the virus removal membrane 10 is, for example, 13 nm or larger and 21 nm or smaller, preferably 13 nm or larger and 20.5 nm or smaller, more preferably 13.5 nm or larger and 20.5 nm or smaller. An average pore size smaller than 13 nm might decrease a filtration rate. An average pore size larger than 21 nm might cause the leakage of viruses. The pore size of holes decreases and in turn increases from the primary side toward the secondary side on the cross section of the virus removal membrane 10. The virus capture site comprises, for example, a site having the smallest pore size of holes on the cross section of the virus removal membrane 10.

Alternatively, the virus removal membrane may comprise at least a site where the pore size decreases from the primary side toward the secondary side on the cross section of the virus removal membrane 10. Alternatively, the pore size may be decrease and in turn become constant from the primary side toward the secondary side on the cross section of the virus removal membrane 10, and a most closely packed layer may be comprised near the secondary-side surface.

The virus logarithmic reduction value (LRV) of the virus removal membrane 10 is, for example, 3.00 or more, preferably 4.00 or more for sufficiently removing viruses by membrane filtration, more preferably 4.50 or more, 5.00 or more, or 6.00 or more. At the virus logarithmic reduction value of 6.00 or more, it is considered that viruses are removed and rarely leak.

The logarithmic reduction value (LRV) of the virus removal membrane 10 for gold colloids having a diameter of 30 nm is, for example, 1.00 or more, preferably 1.20 or more. The logarithmic reduction value of the virus removal membrane 10 for gold colloids having a diameter of 20 nm is, for example, 1.00 or more, preferably 1.20 or more. The logarithmic reduction value of the virus removal membrane 10 for gold colloids having a diameter of 15 nm is, for example, 0.10 or more, preferably 0.15 or more, more preferably 0.20 or more. The logarithmic reduction value of the virus removal membrane 10 for gold colloids having a diameter of 10 nm is, for example, less than 0.10.

The gold colloid capture site comprises, for example, a site having the smallest pore size on the cross section of the virus removal membrane 10.

A bubble point to be measured in the virus removal membrane 10 is, for example, 1.2 MPa or higher and 1.8 MPa or lower. A pure water flux to be measured in the virus removal membrane 10 is 30 L/m2/hrs/0.1 MPa or higher and 120 L/m2/hrs/0.1 MPa or lower, 40 L/m2/hrs/0.1 MPa or higher and 115 L/m2/hrs/0.1 MPa or lower, or 50 L/m2/hrs/0.1 MPa or higher and 110 L/m2/hrs/0.1 MPa or lower.

The virus removal membrane according to the embodiment having the characteristics described above is produced by, for example, a method described below. For the production of a virus removal membrane in a hollow fiber membrane form, first, cellulose is dissolved in a copper ammonia solution to prepare a cellulose copper ammonia solution having a cellulose concentration of, for example, approximately 7.0% by weight or higher and approximately 8.0% by weight or lower. An inorganic salt is added to this solution to prepare a raw spinning solution. The addition of the inorganic salt may be performed before the dissolution of cellulose in a copper ammonia solution. A sodium salt, potassium salt, calcium salt, or magnesium salt of sulfuric acid, sulfurous acid, or carbonic acid can be used as the inorganic salt. Among them, a sodium salt or potassium salt of sulfuric acid or sulfurous acid is preferred, and sodium sulfate or sodium sulfite is more preferred. The amount of the inorganic salt added is 0.02% by weight or larger and 0.90% by weight or smaller, 0.03% by weight or larger and 0.80% by weight or smaller, or 0.04% by weight or larger and 0.70% by weight or smaller.

A solution that contains at least one organic solvent and causes the microphase separation of the cellulose copper ammonia solution is prepared as a coagulating liquid, wherein the organic solvent has no hydroxy group, has a solubility of 10% by weight or higher in an aqueous solution containing 28% by weight of ammonia, and does not swell cellulose. The microphase separation will be described later. For example, the coagulating liquid consists of acetone, ammonia, and water. For the production of a hollow fiber membrane, as mentioned later, an inner-side coagulating liquid and an outer-side coagulating liquid are prepared. The inner-side coagulating liquid has, for example, an acetone concentration of approximately 30% by weight or higher and approximately 50% by weight or lower and an ammonia concentration of approximately 0.5% by weight or higher and approximately 1.0% by weight or lower. The outer-side coagulating liquid has, for example, an acetone concentration of approximately 20% by weight or higher and approximately 40% by weight or lower and an ammonia concentration of approximately 0% by weight or higher and approximately 0.2% by weight or lower.

Next, the raw spinning solution is discharged in a constant amount of 1.5 cc/min or larger and 8.0 cc/min or smaller from an annular double-orifice spinneret. At the same time therewith, the inner-side coagulating liquid is discharged from a central spinning orifice disposed at the central portion of the annular double-orifice spinneret. The discharged raw spinning solution and inner-side coagulating liquid are immediately dipped in the outer-side coagulating liquid in a coagulation bath. In this context, microphase separation occurs in the raw spinning solution by the action of the inner-side and outer-side coagulating liquids. The microphase separation means that a cellulose-rich phase is separated as particles having a diameter of 0.01 to several $\mu$m from a solvent or diluted cellulose phase, dispersed, and stabilized. The microphase separation occurs first at the interfaces of the raw spinning solution to the inner-side and outer-side coagulating liquids and also occurs gradually inside the raw spinning solution. The particles formed by the microphase separation form large particles while repeating collision or fusion. At the same time therewith, the particles are gradually solidified by the action of the coagulating liquids to form a hollow fiber membrane having a polymeric porous structure having a three-dimensional linkage of the particles. The formed hollow fiber membrane is rolled up.

When the coagulation bath is constituted by a narrow tube, the flow rate of the raw spinning solution in the coagulation bath is, for example, 5 m/min or more and 20 m/min or less, 8 m/min or more and 15 m/min or less, or 9 m/min or more and 12 m/min or less. The flow rate of the raw spinning solution in the coagulation bath is equal to the take-up rate (spinning rate) of the hollow fiber membrane to be formed. The flow volume of the outer-side coagulating liquid to be sent to the coagulation bath is, for example, 50 cc/min or more and 500 cc/min or less, 100 cc/min or more and 300 cc/min or less, or 130 cc/min or more and 200 cc/min or less. The flow rate of the outer-side coagulating liquid in the coagulation bath, which is determined by dividing the flow volume of the outer-side coagulating liquid by the cross-sectional area of the narrow tube constituting the coagulation bath is, for example, 1.8 m/min or more and 10.4 m/min or less, 3.2 m/min or more and 7.8 m/min or less, or 3.5 m/min or more and 5.4 m/min or less. The ratio of the flow rate of the outer-side coagulating liquid to the flow rate of the raw spinning solution in the coagulation bath is, for example, 0.32 or more and 0.54 or less or 0.33 or more and 0.53 or less. The respective absolute values of the flow rate of the raw spinning solution and the flow rate of the outer-side coagulating liquid are arbitrary as long as the ratio of the flow rate of the outer-side coagulating liquid to the flow rate of the raw spinning solution falls within the aforementioned range.

The rolled-up hollow fiber membrane is dipped in 2% by weight or higher and 10% by weight or lower of dilute sulfuric acid and subsequently washed with pure water. This regenerates the cellulose. Further, water contained in the hollow fiber membrane is replaced with an organic solvent. Methanol, ethanol, acetone, or the like can be used as the organic solvent. Then, both ends of the hollow fiber membrane bundle are fixed, and the bundle is stretched by 1% to 8% and then dried under reduced pressure of 5 kPa or lower at 30°C or higher and 60°C or lower to obtain the virus removal membrane in a hollow fiber membrane form according to the embodiment.

A virus removal membrane in a flat membrane form is produced by, for example, the following method: an inorganic salt is added to a copper ammonia cellulose solution and mixed therewith to obtain a membrane forming solution. Subsequently, the membrane forming solution is subjected to filtration and degasification. The type of the inorganic salt used is the same as above.

Next, the membrane forming solution is casted onto a support media travelling in a coagulation bath and coagulated. The movement rate of the support media is set to approximately 1.0 to 10.0 m/min. The formed flat membrane is regenerated with an acid, then passed through an additional water bath, pulled out thereof, and then dried using a dryer. In this context, the relationship between the casting rate and the movement rate of the coagulating liquid is appropriately set in order to render the virus capture site or the gold colloid capture site homogeneous and closely packed in the virus removal membrane in a flat membrane form to be produced. Specifically, the ratio of the movement rate of the coagulating liquid to the movement rate of the support media is set to a constant range.

The virus removal membrane in a hollow fiber or flat membrane form produced by the aforementioned method can be used for preparing a filter in which a primary-side space on an inlet side for a liquid to be filtered and a secondary-side space on a filtrate outlet side are partitioned by the membrane.

The purification step by cation-exchange chromatography that is carried out in the present embodiment can be combined with an anion-exchange chromatography step to thereby further improve the purity of a purified product.

In a purification step with a conventional general cation-exchange chromatographic support media, a substance of interest is temporarily adsorbed onto the support media and purified by elution. Therefore, impurities having pI higher than that of the substance of interest and impurities having pI lower than that of the substance of interest can both be removed. On the other hand, the flow-through purification of the present embodiment may have a difficulty in removing impurities having pI lower than that of the substance of interest. However, the impurities having pI lower than that of the substance of interest can be removed by purification with an anion-exchange chromatographic support media. Therefore, the purification step with the cation-exchange chromatographic support media according to the present embodiment can be combined with a purification step with an anion-exchange chromatographic support media to thereby achieve efficient purification while maintaining the conventional property of removing impurities.

Examples of the impurities having pI lower than that of the substance of interest include, but are not particularly limited to, host cell-derived protein (HCP), DNA, and protein A, which is an impurity derived from an affinity chromatography step.

The purification step with an anion-exchange chromatographic support media may be performed before or after the purification step with the cation-exchange chromatographic support media. However, when no additional step is comprised between the purification step using the cation-exchange chromatographic support media and the virus removal step or when the purification step using the cation-exchange chromatographic support media and the virus removal step are performed as a continuous process, the purification step using the anion-exchange chromatographic support media precedes the purification step using the cation-exchange chromatographic support media.

The anion-exchange chromatographic support media is not particularly limited, and any anion-exchange chromatographic support media in a membrane form permits a high flow rate and achieves construction of a more efficient purification step. The anion-exchange chromatographic support media may also have a structure having a graft polymer chain on a substrate.

The structure of the anion-exchange chromatographic support media is not particularly limited, and any structure having a graft polymer on a substrate can sterically adsorb impurities and can therefore be expected to have the higher property of removing impurities.

The purification method with the anion-exchange chromatographic support media is not particularly limited, and flow-through purification achieves more efficient purification.

The amount of antibodies loaded in the anion-exchange chromatography step is not particularly limited as long as impurities can be removed. The amount of antibodies loaded is preferably 0.2 g or larger, more preferably 0.5 g or larger, 1.0 g or larger, 2.0 g or larger, or 4.0 g or larger, per mL of the support media from the viewpoint of efficient purification.

Buffer replacement may or may not be performed between the purification step with the anion-exchange chromatographic support media and the purification step with the cation-exchange chromatographic support media. Without buffer replacement, more efficient purification can be performed.

The purification step with the anion-exchange chromatographic support media and the purification step with the cation-exchange chromatographic support media may be continuously performed, or a purified product may be temporarily stored in a tank after the completion of a preceding step and then subjected to the next step.

pH adjustment may be performed between the purification step with the anion-exchange chromatographic support media and the purification step with the cation-exchange chromatographic support media. The pH adjustment enhances the property of removing impurities having pI around the pH of the former processing. Specifically, in the case of first performing the purification with the cation-exchange chromatographic support media, the pH is increased by the addition of a base before the purification with the anion-exchange chromatographic support media so that a larger amount of impurities can be removed. On the other hand, in the case of first using the anion-exchange chromatographic support media, the pH is decreased by the addition of an acid before the purification with the cation-exchange chromatographic support media so that a larger amount of impurities can be removed. The pH value to be changed is not particularly limited as long as the property of removing impurities is improved. The pH can be arbitrarily changed according to the target impurities. Examples of the value of the amount of change in pH include 0.01, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, and 3.0. The presented values themselves or any value between these values may be selected.

Preferably, the property of removing impurities is particularly improved by changing the pH by 0.1 or more, though this value is not particularly limited as long as impurities can be removed.

As with the pH, electric conductivity adjustment may be performed between the purification step with the cation-exchange chromatographic support media and the purification step with the anion-exchange chromatographic support media. The electric conductivity value to be changed is not particularly limited as long as the property of removing impurities is improved. The electric conductivity can be arbitrarily changed according to the target impurities. Examples of the value of the amount of change in electric conductivity include 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 3.0, 4.0, and 5.0. The presented values themselves or any value between these values may be selected.

An affinity chromatography step may be performed before the anion-exchange chromatography step and the purification step by cation-exchange chromatography. The anion-exchange chromatography step and the flow-through purification by cation-exchange chromatography as described above can be performed after the affinity chromatography step to obtain more highly pure matter of interest.

According to the present embodiment, efficient purification is achieved by performing the flow-through purification by cation-exchange chromatography and the flow-through purification by anion-exchange chromatography without buffer replacement of an antibody-containing eluate after the affinity chromatography step. Such an eluting solution includes a buffer composed mainly of a monovalent acid.

In a general anion-exchange chromatography step, use of an elution buffer composed mainly of a polyvalent acid tends to reduce the amount of substances adsorbed and reduce the property of removing impurities. Therefore, for using an elution buffer composed mainly of a polyvalent acid in an affinity chromatography step, it is desirable that the buffer should be replaced before anion-exchange chromatography. However, provided that elution in the affinity chromatography step is performed using an elution buffer composed mainly of a monovalent acid, purification by the cation-exchange chromatography step and the anion-exchange chromatography step can be performed without the need of buffer replacement.

The elution buffer composed mainly of a monovalent acid is not particularly limited as long as elution is achieved in the affinity chromatography step and impurities can be removed in a subsequent ion-exchange chromatography step. An acetate buffer is desirable.

The elution buffer for use in the elution in the affinity chromatography step has, but not particularly limited to, an electric conductivity of desirably 10.0 mS/cm or lower, more preferably 7.0 mS/cm or lower, further preferably 5.0 mS/cm or lower, particularly preferably 3.0 mS/cm or lower, from the viewpoint of the property of removing impurities in the subsequent flow-through purification by cation-exchange chromatography.

For keeping the electric conductivity of an elution pool low, it is desirable that the support media should be washed with a buffer solution having a low electric conductivity immediately before elution in the affinity chromatography step. The buffer solution can have sufficiently high pH and a sufficiently low electric conductivity so as not to elute antibodies.

Such pH is preferably 5.0 or higher, more preferably 6.0 or higher, further preferably 7.0 or higher, and such electric conductivity is preferably 10.0 mS/cm or lower, more preferably 7.0 mS/cm or lower, further preferably 5.0 mS/cm or lower, particularly preferably 3.0 mS/cm or lower.

After the elution in the affinity chromatography step, virus inactivation may be performed by exposing viruses that may be contained in the eluate to acidic (low pH) conditions for a given time. For the virus inactivation, pH is desirably 4.0 or lower, preferably 3.8 or lower, more preferably 3.6 or lower, further preferably 3.5 or lower, particularly preferably 3.4 or lower.

After the affinity chromatography step or the subsequent virus inactivation, the pH of the eluate may be adjusted by the addition of a base to the eluate. The value of the pH thus adjusted is not particularly limited as long as impurities can be removed in a subsequent ion-exchange chromatography step. The value of the adjust pH is preferably 4.0 or higher, more preferably 5.0 or higher, further preferably 6.0 or higher.

Examples

Hereinafter, the embodiments will be described further specifically with reference to Examples, Comparative Examples, Reference Examples, and Reference Comparative Examples. However, the embodiments are not intended to be limited by these examples by any means.

(Reference Example 1)

In Reference Example 1, a cation-exchange membrane in a hollow fiber form having carboxylic acid groups was synthesized by the radiation graft polymerization method.

1) Radiation graft polymerization

3.09 g of N-isopropylacrylamide, 1.54 g of butyl methacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.31 g of a cation-exchange membrane having a graft ratio of 77%.

The volume of one filament of the obtained hollow fiber was measured and was consequently 1.05 mL. This hollow fiber was hydrophilized with ethanol, followed by replacement with water. After removal of water, 10 mL of a 0.1 mol/L aqueous sodium hydroxide solution was added to the hollow fiber. The hollow fiber was left for 1 hour, and then, the aqueous sodium hydroxide solution was isolated, followed by the addition of 10 mL of pure water. The hollow fiber was further left for 1 hour, and then, sodium hydroxide remaining in the membrane was recovered by recovering pure water. The recovered sodium hydroxide solutions were combined and titrated using 0.1 mol/L hydrochloric acid. As a result, 7.98 mL was required for neutralization. The blank was 9.77 mL, revealing that sodium hydroxide-reacted weak cation-exchange groups carried by the membrane were 179 umol. This value can be divided by the measured volume to determine a weak cation-exchange group density. The cation-exchange group density was 170 mmol/L. The resultant was made into a module (membrane volume: 0.25 mL) to prepare cation-exchange membrane 1 according to Reference Example 1. The mass percentages of cation-exchange group-containing monomers and neutral monomers were 0.100 and 0.900, respectively.

2) Preparation of cell culture solution

A culture supernatant containing 0.115 g/L AE6F4 antibodies (human monoclonal antibodies) as antibody proteins was prepared. AE6F4-producing cells were kindly provided by associate professor Yoshinori Katakura (Faculty of Agriculture, Kyushu University). The AE6F4 antibody-producing cells were cultured with reference to the literature (Proceedings of Annual Meeting of The Society for Biotechnology, Japan, 1994, Vol. 65, p. 65). The culture solution containing the AE6F4 antibody-producing cells was filtered through a filtration membrane (manufactured by Asahi Kasei Medical Co., Ltd., trade name: BioOptimal(R) MF-SL) to obtain an antibody solution (culture supernatant) containing impurities and the antibodies. The filtration was carried out with reference to the instruction manual of the distributor.

3) Purification of antibody protein with protein A column

2 L of the filtered antibody solution was added to a protein A column (manufactured by GE Healthcare Biosciences Corp., packed with MabSelect Sure) equilibrated with 150 mL of a phosphate buffer solution (20 mmol/L sodium phosphate + 150 mmol/L NaCl (pH 8.0)) so that the antibody proteins were adsorbed onto protein A. Next, the column was washed by passing 20 mL of a phosphate buffer solution (20 mmol/L sodium phosphate + 150 mmol/L NaCl (pH 8.0)). Then, the antibody proteins were eluted from the protein A column by passing 240 mL of an elution buffer solution (100 mmol/L sodium citrate (pH 3.6)) to the column to recover an antibody solution with impurities reduced to some extent.

4) Preparation of aggregate

A portion of the obtained antibody solution was adjusted to pH 3 by the addition of hydrochloric acid and left for 1 hour. Then, the solution was neutralized using an aqueous sodium hydroxide solution to prepare an antibody solution containing a large amount of aggregates.

5) Preparation of antibody solution containing aggregate

The antibody solution obtained from the protein A column was buffer-replaced with a 15 mmol/L tris buffer solution (pH 7.0), and the resulting solution was mixed with a solution obtained by the buffer replacement of the antibody solution containing a large amount of aggregates with a 15 mmol/L tris buffer solution (pH 7.0), at an arbitrary ratio to prepare an antibody solution containing aggregates.

6) Measurement of amount of aggregate

The obtained antibody solution was measured using a size exclusion chromatography (SEC) apparatus under the following conditions:

Column: ACQUITY YPLC BEH200 SEC 1.7 um (manufactured by Waters Corp.)
Column temperature: 30°C
System: ACQUITY UPLC H CLASS (manufactured by Waters Corp.)
Mobile phase: aqueous solution of 0.1 mol/L disodium hydrogen phosphate + 0.2 mol/L L(+)-arginine (adjusted to pH 6.7 with hydrochloric acid)

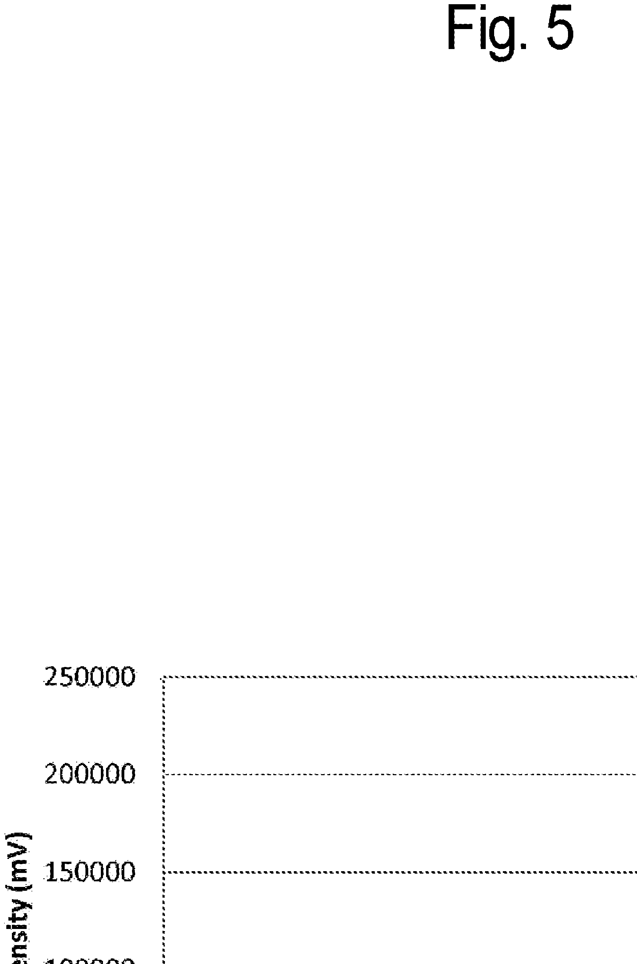

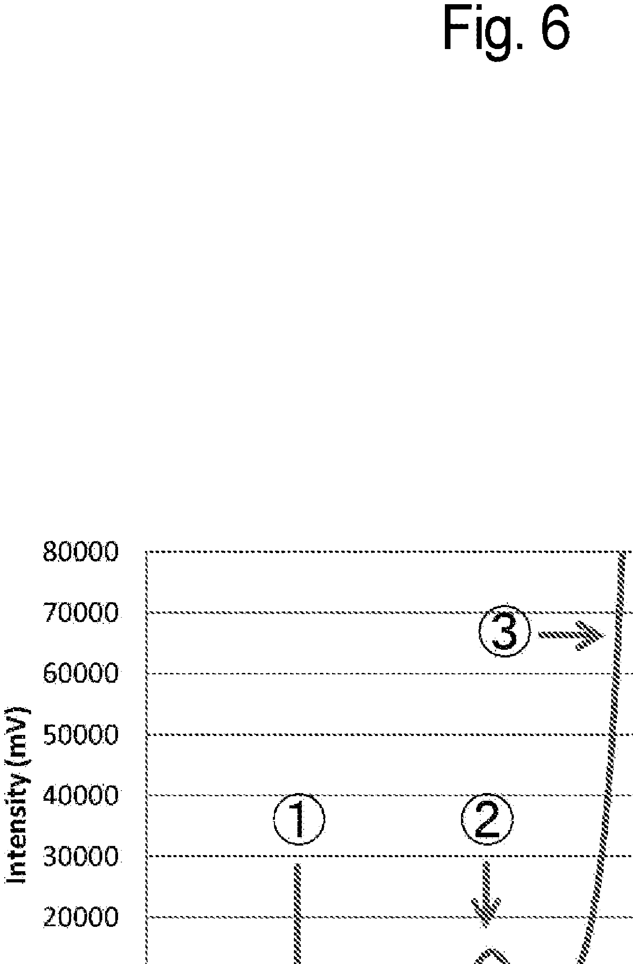

As a result, the chromatographic chart of Figure 5 was obtained. An enlarged view of this chart is shown in Figure 6. Peaks (1) and (2) in Figure 2 depict antibody aggregates, and peak (3) depicts monomers. The percentage of the aggregates (1) calculated from the peak area of the chromatographic chart was 1.54%, the percentage of the aggregates (2) was 2.20%, and the percentage of the monomers was 96.25%. In Reference Examples and Reference Comparative Examples below, the first appearing peak of aggregates is referred to as aggregates (1), and the second appearing peak of aggregates is referred to as aggregates (2). The reduction rate of the percentage of antibody aggregates is indicated by percentage of a value obtained by dividing the difference of the percentage of the aggregate components (1) and (2) after the processing from the percentage of the aggregate components (1) and (2) before the processing by the content of the aggregate components (1) and (2) before the processing.

7) Removal of aggregate

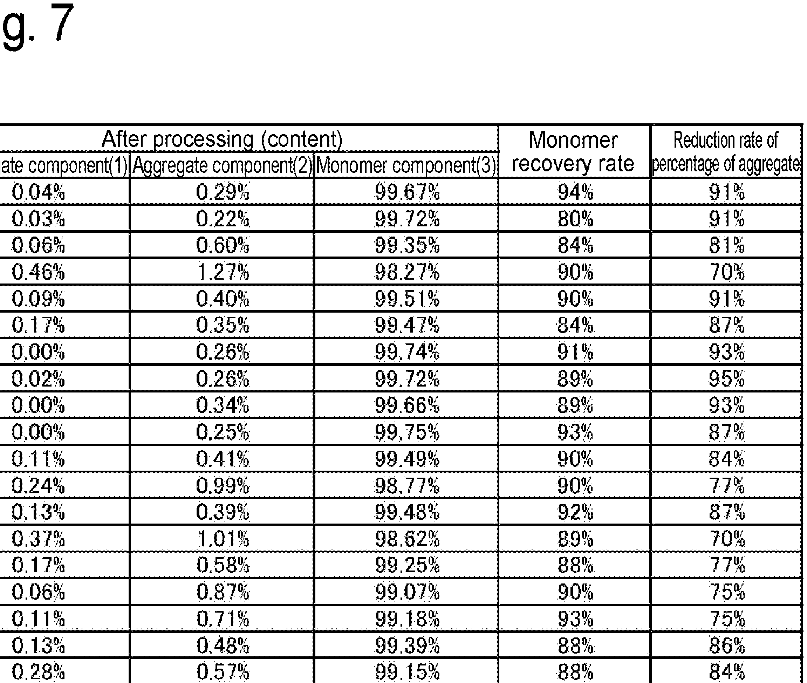

The antibody solution containing aggregate components (impurities) and monomer components (physiologically active substance of interest) of the antibody proteins was contacted with cation-exchange membrane 1. The amount of the antibody solution added was 20 mL (concentration: 5.12 mg/mL), the flow rate was 1.5 mL/min, and the temperature was 25°C. After the flowing of the antibody solution, cation-exchange membrane 1 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7. In Figure 7, "Reduction rate of percentage of aggregate" is indicated by percentage of a value obtained by dividing the difference of the content of the aggregate components (1) and (2) after the processing from the content of the aggregate components (1) and (2) before the processing by the content of the aggregate components (1) and (2) before the processing.

(Reference Example 2)

In Reference Example 2, cation-exchange membrane 2 described below was used. Cation-exchange membrane 2 was synthesized as follows: 3.09 g of N-isopropylacrylamide, 1.03 g of butyl methacrylate, and 1.03 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.32 g of a cation-exchange membrane having a graft ratio of 77%. The cation-exchange group density measured in the same way as in Reference Example 1 was 390 mmol/L. The resultant was made into a module (membrane volume: 0.25 mL) to prepare cation-exchange membrane 2 according to Reference Example 2. The mass percentages of cation-exchange group-containing monomers and neutral monomers were 0.217 and 0.783, respectively.

In the antibody solution used in Reference Example 2, the percentage of the aggregates (1) was 1.59%, the percentage of the aggregates (2) was 1.67%, and the percentage of the monomers was 96.74%.

The antibody solution containing aggregate components (impurities) and monomer components (physiologically active substance of interest) of the antibody proteins was contacted with cation-exchange membrane 2. The amount of the antibody solution added was 20 mL (concentration: 5.29 mg/mL), the flow rate was 1.5 mL/min, and the temperature was 25°C. After the flowing of the antibody solution, cation-exchange membrane 2 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Example 3)

In Reference Example 3, cation-exchange membrane 3 described below was used. Cation-exchange membrane 3 was synthesized as follows: 3.09 g of N-isopropylacrylamide, 1.77 g of butyl methacrylate, and 0.28 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.17 g of a cation-exchange membrane having a graft ratio of 72%. The cation-exchange group density measured in the same way as in Reference Example 1 was 59 mmol/L. The resultant was made into a module (membrane volume: 0.25 mL) to prepare cation-exchange membrane 3 according to Reference Example 3. The mass percentages of cation-exchange group-containing monomers and neutral monomers were 0.035 and 0.965, respectively.

The antibody solution used in Reference Example 3 was a 15 mmol/L tris buffer solution (pH 8.0). The percentage of the aggregates (1) was 1.31%, the percentage of the aggregates (2) was 2.04%, and the percentage of the monomers was 96.66%.

The antibody solution containing aggregate components (impurities) and monomer components (physiologically active substance of interest) of the antibody proteins was contacted with cation-exchange membrane 3. The amount of the antibody solution added was 20 mL (concentration: 5.37 mg/mL), the flow rate was 1.5 mL/min, and the temperature was 25°C. After the flowing of the antibody solution, cation-exchange membrane 3 was washed with 10 mL of a 15 mmol/L tris buffer solution (pH 8.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Example 4)

In Reference Example 4, cation-exchange membrane 3 and an antibody solution described below were used. The antibody solution used in Reference Example 4 was a 15 mmol/L tris buffer solution (pH 9.0). The percentage of the aggregates (1) was 2.49%, the percentage of the aggregates (2) was 3.19%, and the percentage of the monomers was 94.32%.

The antibody solution containing aggregate components (impurities) and monomer components (physiologically active substance of interest) of the antibody proteins was contacted with cation-exchange membrane 3. The amount of the antibody solution added was 20 mL (concentration: 4.31 mg/mL), the flow rate was 1.5 mL/min, and the temperature was 25°C. After the flowing of the antibody solution, cation-exchange membrane 3 was washed with 10 mL of a 15 mmol/L tris buffer solution (pH 9.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Example 5)

In Reference Example 5, cation-exchange membrane 4 described below was used. Cation-exchange membrane 4 was synthesized as follows: 3.83 g of N-isopropylacrylamide, 1.92 g of butyl methacrylate, and 0.64 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.32 g of a cation-exchange membrane having a graft ratio of 77%. The cation-exchange group density measured in the same way as in Reference Example 1 was 183 mmol/L. The resultant was made into a module (membrane volume: 0.25 mL) to prepare cation-exchange membrane 4 according to Reference Example 5. The mass percentages of cation-exchange group-containing monomers and neutral monomers were 0.102 and 0.898, respectively.

The antibody solution used in Reference Example 5 was a 15 mmol/L tris buffer solution (pH 7.0). The percentage of the aggregates (1) was 3.25%, the percentage of the aggregates (2) was 2.07%, and the percentage of the monomers was 94.68%.

The antibody solution containing aggregate components (impurities) and monomer components (physiologically active substance of interest) of the antibody proteins was contacted with cation-exchange membrane 4. The amount of the antibody solution added was 25 mL (concentration: 5.76 mg/mL), the flow rate was 1.5 mL/min, and the temperature was 25°C. After the flowing of the antibody solution, cation-exchange membrane 4 was washed with 10 mL of a 15 mmol/L tris buffer solution (pH 7.0) of 25°C flowing at a flow rate of 1.5 mL/min. 35 mL of a solution was recovered by the flow-through step and the washing step. The recovered solution was applied to size exclusion chromatography (SEC). As a result, the content of the aggregate components was decreased. The results are shown in Figure 7.

(Reference Example 6)

In Reference Example 6, cation-exchange membrane 5 described below was used. Cation-exchange membrane 5 was synthesized as follows: 1.49 g of 2-hydroxyethyl methacrylate, 0.72 g of butyl methacrylate, and 0.36 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 4.35 g of a cation-exchange membrane having a graft ratio of 45%. The cation-exchange group density measured in the same way as in Reference Example 1 was 155 mmol/L. The resultant was made into a module (membrane volume: 0.25 mL) to prepare cation-exchange membrane 5 according to Reference Example 6. The mass percentages of cation-exchange group-containing monomers and neutral monomers were 0.148 and 0.852, respectively.

The antibody solution used in Reference Example 6 was a 15 mmol/L tris buffer solution (pH 7.0). The percentage of the aggregates (1) was 2.35%, the percentage of the aggregates (2) was 1.82%, and the percentage of the monomers was 95.83%.

The antibody solution containing aggregate components (impurities) and monomer components (physiologically active substance of interest) of the antibody proteins was contacted with cation-exchange membrane 5. The amount of the antibody solution added was 20 mL (concentration: 4.73 mg/mL), the flow rate was 1.5 mL/min, and the temperature was 25°C. After the flowing of the antibody solution, cation-exchange membrane 5 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Example 7)

In Reference Example 7, cation-exchange membrane 6 described below was used. Cation-exchange membrane 6 was synthesized as follows: 2.06 g of 2-hydroxyethyl methacrylate, 1.03 g of butyl methacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 4.72 g of a cation-exchange membrane having a graft ratio of 57%. The cation-exchange group density measured in the same way as in Reference Example 1 was 178 mmol/L. The resultant was made into a module (membrane volume: 0.25 mL) to prepare cation-exchange membrane 6 according to Reference Example 7. The mass percentages of cation-exchange group-containing monomers and neutral monomers were 0.134 and 0.866, respectively.

The antibody solution used in Reference Example 7 was a 15 mmol/L tris buffer solution (pH 7.0). The percentage of the aggregates (1) was 2.12%, the percentage of the aggregates (2) was 1.56%, and the percentage of the monomers was 96.32%.

The antibody solution containing aggregate components (impurities) and monomer components (physiologically active substance of interest) of the antibody proteins was contacted with cation-exchange membrane 6. The amount of the antibody solution added was 25 mL (concentration: 5.17 mg/mL), the flow rate was 1.5 mL/min, and the temperature was 25°C. After the flowing of the antibody solution, cation-exchange membrane 6 was washed with 10 mL of a 15 mmol/L tris buffer solution (pH 7.0) of 25°C flowing at a flow rate of 1.5 mL/min. 35 mL of a solution was recovered by the flow-through step and the washing step. The recovered solution was applied to size exclusion chromatography (SEC). As a result, the content of the aggregate components was decreased. The results are shown in Figure 7.

(Reference Example 8)

In Reference Example 8, cation-exchange membrane 7 described below was used. Cation-exchange membrane 7 was synthesized as follows: 2.57 g of 2-hydroxyethyl methacrylate, 1.29 g of butyl methacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.28 g of a cation-exchange membrane having a graft ratio of 76%. The cation-exchange group density measured in the same way as in Reference Example 1 was 177 mmol/L. The resultant was made into a module (membrane volume: 0.25 mL) to prepare cation-exchange membrane 7 according to Reference Example 8. The mass percentages of cation-exchange group-containing monomers and neutral monomers were 0.100 and 0.900, respectively.

The antibody solution used in Reference Example 8 was a 15 mmol/L tris buffer solution (pH 7.0). The percentage of the aggregates (1) was 3.37%, the percentage of the aggregates (2) was 1.94%, and the percentage of the monomers was 94.69%.

The antibody solution containing aggregate components (impurities) and monomer components (physiologically active substance of interest) of the antibody proteins was contacted with cation-exchange membrane 7. The amount of the antibody solution added was 25 mL (concentration: 5.49 mg/mL), the flow rate was 1.5 mL/min, and the temperature was 25°C. After the flowing of the antibody solution, cation-exchange membrane 7 was washed with 10 mL of a 15 mmol/L tris buffer solution (pH 7.0) of 25°C flowing at a flow rate of 1.5 mL/min. 35 mL of a solution was recovered by the flow-through step and the washing step. The recovered solution was applied to size exclusion chromatography (SEC). As a result, the content of the aggregate components was decreased. The results are shown in Figure 7.

(Reference Example 9)

In Reference Example 9, cation-exchange membrane 8 described below was used. Cation-exchange membrane 8 was synthesized as follows: 2.57 g of 2-hydroxyethyl methacrylate, 1.29 g of butyl methacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 4.97 g of a cation-exchange membrane having a graft ratio of 66%. The cation-exchange group density measured in the same way as in Reference Example 1 was 174 mmol/L. The resultant was made into a module (membrane volume: 0.25 mL) to prepare cation-exchange membrane 8 according to Reference Example 9. The mass percentages of cation-exchange group-containing monomers and neutral monomers were 0.114 and 0.886, respectively.

The antibody solution used in Reference Example 9 was a 15 mmol/L tris buffer solution (pH 7.0). The percentage of the aggregates (1) was 2.45%, the percentage of the aggregates (2) was 2.39%, and the percentage of the monomers was 95.17%.

The antibody solution containing aggregate components (impurities) and monomer components (physiologically active substance of interest) of the antibody proteins was contacted with cation-exchange membrane 8. The amount of the antibody solution added was 25 mL (concentration: 6.02 mg/mL), the flow rate was 1.5 mL/min, and the temperature was 25°C. After the flowing of the antibody solution, cation-exchange membrane 8 was washed with 10 mL of a 15 mmol/L tris buffer solution (pH 7.0) of 25°C flowing at a flow rate of 1.5 mL/min. 35 mL of a solution was recovered by the flow-through step and the washing step. The recovered solution was applied to size exclusion chromatography (SEC). As a result, the content of the aggregate components was decreased. The results are shown in Figure 7.

(Reference Example 10)

In Reference Example 10, cation-exchange membrane 9 described below was used. Cation-exchange membrane 9 was synthesized as follows: 2.40 g of 2-hydroxyethyl methacrylate, 1.20 g of butyl methacrylate, and 0.77 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 4.82 g of a cation-exchange membrane having a graft ratio of 61%. The cation-exchange group density measured in the same way as in Reference Example 1 was 249 mmol/L. The resultant was made into a module (membrane volume: 0.25 mL) to prepare cation-exchange membrane 9 according to Reference Example 10. The mass percentages of cation-exchange group-containing monomers and neutral monomers were 0.177 and 0.823, respectively.

The antibody solution used in Reference Example 10 was a 15 mmol/L tris buffer solution (pH 7.0). The percentage of the aggregates (1) was 0.72%, the percentage of the aggregates (2) was 1.14%, and the percentage of the monomers was 98.14%.

The antibody solution containing aggregate components (impurities) and monomer components (physiologically active substance of interest) of the antibody proteins was contacted with cation-exchange membrane 9. The amount of the antibody solution added was 50 mL (concentration: 5.30 mg/mL), the flow rate was 1.5 mL/min, and the temperature was 25°C. After the flowing of the antibody solution, cation-exchange membrane 9 was washed with 10 mL of a 15 mmol/L tris buffer solution (pH 7.0) of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 11)

In Reference Example 11, cation-exchange membrane 10 described below was used. Cation-exchange membrane 10 was synthesized as follows: 3.08 g of 2-hydroxyethyl methacrylate, 1.54 g of butyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.31 g of a cation-exchange membrane having a graft ratio of 77%. The cation-exchange group density measured in the same way as in Reference Example 1 was 175 mmol/L. The resultant was made into a module (membrane volume: 0.25 mL) to prepare cation-exchange membrane 10 according to Reference Example 11. The mass percentages of cation-exchange group-containing monomers and neutral monomers were 0.103 and 0.897, respectively.

The antibody solution used in Reference Example 11 was a 15 mmol/L tris buffer solution (pH 7.0). The percentage of the aggregates (1) was 1.57%, the percentage of the aggregates (2) was 1.55%, and the percentage of the monomers was 96.88%.

The antibody solution containing aggregate components (impurities) and monomer components (physiologically active substance of interest) of the antibody proteins was contacted with cation-exchange membrane 10. The amount of the antibody solution added was 40 mL (concentration: 5.91 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7.0) of 25°C flowing at a flow rate of 1.5 mL/min. 50 mL of a solution was recovered by the flow-through step and the washing step. The recovered solution was applied to size exclusion chromatography (SEC). As a result, the content of the aggregate components was decreased. The results are shown in Figure 7.

(Reference Example 12)

In Reference Example 12, cation-exchange membrane 11 described below was used. Cation-exchange membrane 11 was synthesized as follows: 1.93 g of 2-hydroxyethyl methacrylate, 1.93 g of butyl methacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.06 g of a cation-exchange membrane having a graft ratio of 69%. The cation-exchange group density measured in the same way as in Reference Example 1 was 177 mmol/L. The resultant was made into a module (membrane volume: 0.25 mL) to prepare cation-exchange membrane 11 according to Reference Example 12. The mass percentages of cation-exchange group-containing monomers and neutral monomers were 0.11 and 0.89, respectively.

The antibody solution used in Reference Example 12 was a 15 mmol/L tris buffer solution (pH 7.0). The percentage of the aggregates (1) was 2.71%, the percentage of the aggregates (2) was 2.69%, and the percentage of the monomers was 94.60%.

The antibody solution containing aggregate components (impurities) and monomer components (physiologically active substance of interest) of the antibody proteins was contacted with cation-exchange membrane 11. The amount of the antibody solution added was 30 mL (concentration: 5.08 mg/mL), the flow rate was 1.5 mL/min, and the temperature was 25°C. After the flowing of the antibody solution, cation-exchange membrane 11 was washed with 10 mL of a 15 mmol/L tris buffer solution (pH 7.0) of 25°C flowing at a flow rate of 1.5 mL/min. 40 mL of a solution was recovered by the flow-through step and the washing step. The recovered solution was applied to size exclusion chromatography (SEC). As a result, the content of the aggregate components was decreased. The results are shown in Figure 7.

(Reference Example 13)

In Reference Example 13, cation-exchange membrane 10 and an antibody solution described below were used. The antibody solution used in Reference Example 13 was a 15 mmol/L tris buffer solution (pH 7.0) containing 10 mmol/L sodium chloride. The percentage of the aggregates (1) was 2.47%, the percentage of the aggregates (2) was 1.59%, and the percentage of the monomers was 95.94%.

The antibody solution containing aggregate components (impurities) and monomer components (physiologically active substance of interest) of the antibody proteins was contacted with cation-exchange membrane 10. The amount of the antibody solution added was 50 mL (concentration: 5.31 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7.0) containing 10 mmol/L sodium chloride of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 14)

In Reference Example 14, cation-exchange membrane 10 and an antibody solution described below were used. The antibody solution used in Reference Example 14 was a 15 mmol/L tris buffer solution (pH 7.0) containing 30 mmol/L sodium chloride. The percentage of the aggregates (1) was 2.32%, the percentage of the aggregates (2) was 2.21%, and the percentage of the monomers was 95.47%.

The antibody solution containing aggregate components (impurities) and monomer components (physiologically active substance of interest) of the antibody proteins was contacted with cation-exchange membrane 10. The amount of the antibody solution added was 50 mL (concentration: 5.66 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7.0) containing 30 mmol/L sodium chloride of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 15)

In Reference Example 15, cation-exchange membrane 10 and an antibody solution described below were used. The antibody solution used in Reference Example 15 was a 15 mmol/L tris buffer solution (pH 7.5). The percentage of the aggregates (1) was 1.32%, the percentage of the aggregates (2) was 2.00%, and the percentage of the monomers was 96.68%.

The antibody solution containing aggregate components (impurities) and monomer components (physiologically active substance of interest) of the antibody proteins was contacted with cation-exchange membrane 10. The amount of the antibody solution added was 50 mL (concentration: 5.36 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7.5) of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 16)

In Reference Example 16, cation-exchange membrane 10 and an antibody solution described below were used. The antibody solution used in Reference Example 16 was a 15 mmol/L tris buffer solution (pH 7.5) containing 10 mmol/L sodium chloride. The percentage of the aggregates (1) was 1.59%, the percentage of the aggregates (2) was 2.20%, and the percentage of the monomers was 96.21%.

The antibody solution containing aggregate components (impurities) and monomer components (physiologically active substance of interest) of the antibody proteins was contacted with cation-exchange membrane 10. The amount of the antibody solution added was 50 mL (concentration: 5.63 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7.5) containing 10 mmol/L sodium chloride of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 17)

In Reference Example 17, cation-exchange membrane 10 and an antibody solution described below were used. The antibody solution used in Reference Example 17 was a 15 mmol/L tris buffer solution (pH 8). The percentage of the aggregates (1) was 1.41%, the percentage of the aggregates (2) was 1.81%, and the percentage of the monomers was 96.78%.

The antibody solution containing aggregate components (impurities) and monomer components (physiologically active substance of interest) of the antibody proteins was contacted with cation-exchange membrane 10. The amount of the antibody solution added was 50 mL (concentration: 5.30 mg/mL), the flow rate was 1.5 mL/min, and the temperature was 25°C. After the flowing of the antibody solution, cation-exchange membrane 10 was washed with 10 mL of a 15 mmol/L tris buffer solution (pH 8) of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 18)

In Reference Example 18, cation-exchange membrane 10 and an antibody solution described below were used. The antibody solution used in Reference Example 18 was a 15 mmol/L phosphate buffer solution (pH 7.0). The percentage of the aggregates (1) was 2.30%, the percentage of the aggregates (2) was 2.08%, and the percentage of the monomers was 95.62%.

The antibody solution containing aggregate components (impurities) and monomer components (physiologically active substance of interest) of the antibody proteins was contacted with cation-exchange membrane 10. The amount of the antibody solution added was 50 mL (concentration: 5.72 mg/mL), the flow rate was 1.5 mL/min, and the temperature was 25°C. After the flowing of the antibody solution, cation-exchange membrane 10 was washed with 10 mL of a 15 mmol/L phosphate buffer solution (pH 7.0) of 25°C flowing at a flow rate of 1.5 mL/min. 60 mL of a solution was recovered by the flow-through step and the washing step. The recovered solution was applied to size exclusion chromatography (SEC). As a result, the content of the aggregate components was decreased. The results are shown in Figure 7.

(Reference Example 19)

In Reference Example 19, cation-exchange membrane 12 described below was used. Cation-exchange membrane 12 was synthesized as follows: 2.57 g of 2-hydroxyethyl methacrylate, 1.29 g of ethylene glycol dimethacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.11 g of a cation-exchange membrane having a graft ratio of 70%. The cation-exchange group density measured in the same way as in Reference Example 1 was 178 mmol/L. The resultant was made into a module (membrane volume: 0.25 mL) to prepare cation-exchange membrane 12 according to Reference Example 19. The mass percentages of cation-exchange group-containing monomers and neutral monomers were 0.109 and 0.891, respectively.

The antibody solution used in Reference Example 19 was a 15 mmol/L tris buffer solution (pH 7.0). The percentage of the aggregates (1) was 2.99%, the percentage of the aggregates (2) was 2.31%, and the percentage of the monomers was 94.70%.

The antibody solution containing aggregate components (impurities) and monomer components (physiologically active substance of interest) of the antibody proteins was contacted with cation-exchange membrane 12. The amount of the antibody solution added was 22 mL (concentration: 5.33 mg/mL), the flow rate was 1.5 mL/min, and the temperature was 25°C. After the flowing of the antibody solution, cation-exchange membrane 12 was washed with 10 mL of a 15 mmol/L tris buffer solution (pH 7.0) of 25°C flowing at a flow rate of 1.5 mL/min. 32 mL of a solution was recovered by the flow-through step and the washing step. The recovered solution was applied to size exclusion chromatography (SEC). As a result, the content of the aggregate components was decreased. The results are shown in Figure 7.

(Reference Example 20)

In Reference Example 20, cation-exchange membrane 13 described below was used. Cation-exchange membrane 13 was synthesized as follows: 2.69 g of 2-hydroxyethyl methacrylate, 1.17 g of ethylene glycol dimethacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.16 g of a cation-exchange membrane having a graft ratio of 70%. The cation-exchange group density measured in the same way as in Reference Example 1 was 176 mmol/L. The resultant was made into a module (membrane volume: 0.25 mL) to prepare cation-exchange membrane 13 according to Reference Example 20. The mass percentages of cation-exchange group-containing monomers and neutral monomers were 0.105 and 0.895, respectively.

The antibody solution used in Reference Example 20 was a 15 mmol/L tris buffer solution (pH 7.0). The percentage of the aggregates (1) was 2.48%, the percentage of the aggregates (2) was 2.24%, and the percentage of the monomers was 95.29%.

The antibody solution containing aggregate components (impurities) and monomer components (physiologically active substance of interest) of the antibody proteins was contacted with cation-exchange membrane 13. The amount of the antibody solution added was 22 mL (concentration: 5.70 mg/mL), the flow rate was 1.5 mL/min, and the temperature was 25°C. After the flowing of the antibody solution, cation-exchange membrane 13 was washed with 10 mL of a 15 mmol/L tris buffer solution (pH 7.0) of 25°C flowing at a flow rate of 1.5 mL/min. 32 mL of a solution was recovered by the flow-through step and the washing step. The recovered solution was applied to size exclusion chromatography (SEC). As a result, the content of the aggregate components was decreased. The results are shown in Figure 7.

(Reference Example 21)

In Reference Example 21, cation-exchange membrane 14 described below was used. Cation-exchange membrane 14 was synthesized as follows: 2.50 g of 2- hydroxyethyl methacrylate, 1.36 g of diethylene glycol dimethacrylate, and 0.51 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.16 g of a cation-exchange membrane having a graft ratio of 70%. The cation-exchange group density measured in the same way as in Reference Example 1 was 176 mmol/L. The resultant was made into a module (membrane volume: 0.25 mL) to prepare cation-exchange membrane 14 according to Reference Example 21. The mass percentages of cation-exchange group-containing monomers and neutral monomers were 0.105 and 0.895, respectively.

The antibody solution used in Reference Example 21 was a 15 mmol/L tris buffer solution (pH 7.0). The percentage of the aggregates (1) was 2.73%, the percentage of the aggregates (2) was 2.10%, and the percentage of the monomers was 95.16%.

The antibody solution containing aggregate components (impurities) and monomer components (physiologically active substance of interest) of the antibody proteins was contacted with cation-exchange membrane 14. The amount of the antibody solution added was 40 mL (concentration: 5.52 mg/mL), the flow rate was 1.5 mL/min, and the temperature was 25°C. After the flowing of the antibody solution, cation-exchange membrane 14 was washed with 10 mL of a 15 mmol/L tris buffer solution (pH 7.0) of 25°C flowing at a flow rate of 1.5 mL/min. 50 mL of a solution was recovered by the flow-through step and the washing step. The recovered solution was applied to size exclusion chromatography (SEC). As a result, the content of the aggregate components was decreased. The results are shown in Figure 7.

(Reference Example 22)

In Reference Example 22, cation-exchange membrane 15 described below was used. Cation-exchange membrane 15 was synthesized as follows: 3.08 g of 2-hydroxyethyl methacrylate, 1.54 g of butyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.45 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 8.36 g of a cation-exchange membrane having a graft ratio of 46%. The cation-exchange group density measured in the same way as in Reference Example 1 was 227 mmol/L. The resultant was made into a module (membrane volume: 0.11 mL) to prepare cation-exchange membrane 15 according to Reference Example 22. The mass percentages of cation-exchange group-containing monomers and neutral monomers were 0.115 and 0.885, respectively.

The antibody solution used in Reference Example 22 was a 15 mmol/L tris buffer solution (pH 7.0). The percentage of the aggregates (1) was 3.14%, the percentage of the aggregates (2) was 2.03%, and the percentage of the monomers was 94.82%.

The antibody solution containing aggregate components (impurities) and monomer components (physiologically active substance of interest) of the antibody proteins was contacted with cation-exchange membrane 15. The amount of the antibody solution added was 10 mL (concentration: 5.53 mg/mL), the flow rate was 0.7 mL/min, and the temperature was 25°C. After the flowing of the antibody solution, cation-exchange membrane 15 was washed with 5 mL of a 15 mmol/L tris buffer solution (pH 7.0) of 25°C flowing at a flow rate of 0.7 mL/min. 15 mL of a solution was recovered by the flow-through step and the washing step. The recovered solution was applied to size exclusion chromatography (SEC). As a result, the content of the aggregate components was decreased. The results are shown in Figure 7.

(Reference Example 23)

In Reference Example 23, cation-exchange membrane 16 described below was used. Cation-exchange membrane 16 was synthesized as follows: 3.85 g of 2-hydroxyethyl methacrylate, 0.77 g of butyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.45 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 8.26 g of a cation-exchange membrane having a graft ratio of 44%. The cation-exchange group density measured in the same way as in Reference Example 1 was 198 mmol/L. The resultant was made into a module (membrane volume: 0.11 mL) to prepare cation-exchange membrane 16 according to Reference Example 23. The mass percentages of cation-exchange group-containing monomers and neutral monomers were 0.104 and 0.896, respectively.

The antibody solution used in Reference Example 23 was a 15 mmol/L tris buffer solution (pH 7.0). The percentage of the aggregates (1) was 4.41%, the percentage of the aggregates (2) was 2.16%, and the percentage of the monomers was 93.43%.

The antibody solution containing aggregate components (impurities) and monomer components (physiologically active substance of interest) of the antibody proteins was contacted with cation-exchange membrane 16. The amount of the antibody solution added was 18 mL (concentration: 5.53 mg/mL), the flow rate was 0.7 mL/min, and the temperature was 25°C. After the flowing of the antibody solution, cation-exchange membrane 16 was washed with 5 mL of a 15 mmol/L tris buffer solution (pH 7.0) of 25°C flowing at a flow rate of 0.7 mL/min. 23 mL of a solution was recovered by the flow-through step and the washing step. The recovered solution was applied to size exclusion chromatography (SEC). As a result, the content of the aggregate components was decreased. The results are shown in Figure 7.

(Reference Example 24)

In Reference Example 24, cation-exchange membrane 17 described below was used. Cation-exchange membrane 17 was synthesized as follows: 4.62 g of 2-hydroxyethyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.45 um was placed in a closed container, and the inside air of the container was replaced with nitrogen. Then, the container was cooled with dry ice from outside while irradiated with 25 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 7.99 g of a cation-exchange membrane having a graft ratio of 39%. The cation-exchange group density measured in the same way as in Reference Example 1 was 214 mmol/L. The resultant was made into a module (membrane volume: 0.11 mL) to prepare cation-exchange membrane 17 according to Reference Example 24. The mass percentages of cation-exchange group-containing monomers and neutral monomers were 0.126 and 0.874, respectively.

The antibody solution used in Reference Example 24 was a 15 mmol/L tris buffer solution (pH 7.0). The percentage of the aggregates (1) was 2.42%, the percentage of the aggregates (2) was 1.77%, and the percentage of the monomers was 95.81%.

The antibody solution containing aggregate components (impurities) and monomer components (physiologically active substance of interest) of the antibody proteins was contacted with cation-exchange membrane 17. The amount of the antibody solution added was 18 mL (concentration: 5.64 mg/mL), the flow rate was 0.2 mL/min, and the temperature was 25°C. After the flowing of the antibody solution, cation-exchange membrane 17 was washed with 5 mL of a 15 mmol/L tris buffer solution (pH 7.0) of 25°C flowing at a flow rate of 0.2 mL/min. 23 mL of a solution was recovered by the flow-through step and the washing step. The recovered solution was applied to size exclusion chromatography (SEC). As a result, the content of the aggregate components was decreased. The results are shown in Figure 7.

(Reference Example 25)

In Reference Example 25, cation-exchange membrane 18 described below was used. Cation-exchange membrane 18 was synthesized as follows: 3.85 g of 2-hydroxyethyl methacrylate, 0.77 g of butyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.65 um was placed in a closed container, and the inside air of the container was replaced with nitrogen. Then, the container was cooled with dry ice from outside while irradiated with 25 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 8.29 g of a cation-exchange membrane having a graft ratio of 43%. The cation-exchange group density measured in the same way as in Reference Example 1 was 209 mmol/L. The resultant was made into a module (membrane volume: 0.11 mL) to prepare cation-exchange membrane 18 according to Reference Example 25. The mass percentages of cation-exchange group-containing monomers and neutral monomers were 0.108 and 0.892, respectively.

The antibody solution used in Reference Example 25 was a 15 mmol/L tris buffer solution (pH 7.0). The percentage of the aggregates (1) was 2.96%, the percentage of the aggregates (2) was 2.01%, and the percentage of the monomers was 95.03%.

The antibody solution containing aggregate components (impurities) and monomer components (physiologically active substance of interest) of the antibody proteins was contacted with cation-exchange membrane 18. The amount of the antibody solution added was 18 mL (concentration: 5.61 mg/mL), the flow rate was 0.7 mL/min, and the temperature was 25°C. After the flowing of the antibody solution, cation-exchange membrane 18 was washed with 5 mL of a 15 mmol/L tris buffer solution (pH 7.0) of 25°C flowing at a flow rate of 0.7 mL/min. 23 mL of a solution was recovered by the flow-through step and the washing step. The recovered solution was applied to size exclusion chromatography (SEC). As a result, the content of the aggregate components was decreased. The results are shown in Figure 7.

(Reference Example 26)

In Reference Example 26, cation-exchange membrane 19 described below was used. Cation-exchange membrane 19 was synthesized as follows: 4.62 g of 2-hydroxyethyl methacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.65 um was placed in a closed container, and the inside air of the container was replaced with nitrogen. Then, the container was cooled with dry ice from outside while irradiated with 25 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 8.17 g of a cation-exchange membrane having a graft ratio of 41%. The cation-exchange group density measured in the same way as in Reference Example 1 was 223 mmol/L. The resultant was made into a module (membrane volume: 0.11 mL) to prepare cation-exchange membrane 19 according to Reference Example 26. The mass percentages of cation-exchange group-containing monomers and neutral monomers were 0.127 and 0.873, respectively.

The antibody solution used in Reference Example 26 was a 15 mmol/L tris buffer solution (pH 7.0). The percentage of the aggregates (1) was 2.83%, the percentage of the aggregates (2) was 1.96%, and the percentage of the monomers was 95.21%.

The antibody solution containing aggregate components (impurities) and monomer components (physiologically active substance of interest) of the antibody proteins was contacted with cation-exchange membrane 19. The amount of the antibody solution added was 18 mL (concentration: 5.69 mg/mL), the flow rate was 0.7 mL/min, and the temperature was 25°C. After the flowing of the antibody solution, cation-exchange membrane 19 was washed with 5 mL of a 15 mmol/L tris buffer solution (pH 7.0) of 25°C flowing at a flow rate of 0.7 mL/min. 23 mL of a solution was recovered by the flow-through step and the washing step. The recovered solution was applied to size exclusion chromatography (SEC). As a result, the content of the aggregate components was decreased. The results are shown in Figure 7.

(Reference Example 27)

In Reference Example 27, cation-exchange membrane 20 described below was used. Cation-exchange membrane 20 was synthesized as follows: 2.99 g of 2-hydroxyethyl methacrylate, 1.63 g of diethylene glycol dimethacrylate, and 0.57 g of methacrylic acid were dissolved in 240 mL of an aqueous solution containing 50% by volume of t-butyl alcohol, and the solution was used as a reaction solution after nitrogen bubbling for 30 minutes. 5.80 g (15 cm, 30 filaments) of polyvinylidene fluoride porous hollow fiber having an outer diameter of 2.0 mm, an inner diameter of 1.1 mm, and an average pore size of 0.65 um was placed in a closed container, and the inside air of the container was replaced with nitrogen. Then, the container was cooled with dry ice from outside while irradiated with 25 kGy of $\gamma$ ray to generate radicals.

The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 8.42 g of a cation-exchange membrane having a graft ratio of 45%. The cation-exchange group density measured in the same way as in Reference Example 1 was 241 mmol/L. The resultant was made into a module (membrane volume: 0.11 mL) to prepare cation-exchange membrane 20 according to Reference Example 27. The mass percentages of cation-exchange group-containing monomers and neutral monomers were 0.119 and 0.881, respectively.

The antibody solution used in Reference Example 27 was a 15 mmol/L tris buffer solution (pH 7.0). The percentage of the aggregates (1) was 2.54%, the percentage of the aggregates (2) was 1.93%, and the percentage of the monomers was 95.53%.

The antibody solution containing aggregate components (impurities) and monomer components (physiologically active substance of interest) of the antibody proteins was contacted with cation-exchange membrane 20. The amount of the antibody solution added was 18 mL (concentration: 5.51 mg/mL), the flow rate was 0.7 mL/min, and the temperature was 25°C. After the flowing of the antibody solution, cation-exchange membrane 20 was washed with 5 mL of a 15 mmol/L tris buffer solution (pH 7.0) of 25°C flowing at a flow rate of 0.7 mL/min. 23 mL of a solution was recovered by the flow-through step and the washing step. The recovered solution was applied to size exclusion chromatography (SEC). As a result, the content of the aggregate components was decreased. The results are shown in Figure 7.

(Reference Example 28)

In Reference Example 28, cation-exchange membrane 21 described below was used. Cation-exchange membrane 21 was synthesized as follows: 3.09 g of N-isopropylacrylamide, 1.29 g of butyl methacrylate, 0.51 g of glycidyl methacrylate, and 0.26 g of t-butyl methacrylate were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.12 g of a cation-exchange membrane precursor having a graft ratio of 71%.

The hollow fiber containing the graft chain introduced by the radiation graft polymerization method was added to 200 g of a mixed aqueous solution of sodium sulfite and IPA (sodium sulfite/IPA/pure water = 10/15/75 wt%) and reacted at 80°C for 24 hours to convert the epoxy groups in the graft chain to sulfonic acid groups. The hollow fiber thus reacted was washed with pure water. Then, this hollow fiber was added into 0.5 mol/L sulfuric acid and reacted at 80°C for 2 hours to convert the remaining epoxy groups in the graft chain to diol groups. Further, t-butyl groups were deprotected through reaction with 4 mL of methanesulfonic acid in 140 mL of chloroform and converted to carboxyl groups.

The membrane volume and the total cation-exchange group density were measured in the same way as in Reference Example 1 and were consequently 1.0 mL and 49 mmol/L, respectively.

The sulfonic acid group density was determined by the following method: all sulfonic acid groups were hydrogenated by dipping in 1 mol/L hydrochloric acid for 1 hour. Hydrochloric acid was removed by washing with pure water, and then, hydrogen chloride was eluted by the addition of 10 mL of a 1 mol/L aqueous sodium chloride solution. The membrane was left for 1 hour, and then, the aqueous sodium chloride solution containing hydrogen chloride was recovered. 10 mL of a 1 mol/L aqueous sodium chloride solution was further added thereto. The membrane was left for 1 hour, and hydrogen chloride remaining in the membrane was recovered by recovering the solution. The recovered products were combined and titrated using a 0.01 mol/L aqueous sodium hydroxide solution. As a result, 2.09 mL was required for neutralization. The blank was 0.29 mL, revealing that sulfonic acid groups carried by the membrane were 18 umol. This value was divided by the membrane volume to confirm that the density was 18 mmol/L. The carboxyl group density can be determined by subtracting the sulfonic acid group density from the total cation-exchange group density and was 31 mmol/L. The resultant was made into a module (membrane volume: 0.25 mL) to prepare cation-exchange membrane 21 according to Reference Example 28. The mass percentages of cation-exchange group-containing monomers and neutral monomers were 0.047 and 0.953, respectively.

The antibody solution used in Reference Example 28 was a 15 mmol/L acetate buffer solution (pH 6.0). The percentage of the aggregates (1) was 2.80%, the percentage of the aggregates (2) was 3.21%, and the percentage of the monomers was 93.99%.

The antibody solution containing aggregate components (impurities) and monomer components (physiologically active substance of interest) of the antibody proteins was contacted with cation-exchange membrane 21. The amount of the antibody solution added was 20 mL (concentration: 4.99 mg/mL), the flow rate was 1.5 mL/min, and the temperature was 25°C. After the flowing of the antibody solution, cation-exchange membrane 21 was washed with 10 mL of a 15 mmol/L acetate buffer solution (pH 6.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Example 29)

In Reference Example 29, cation-exchange membrane 21 and an antibody solution described below were used. The antibody solution used in Reference Example 29 was a 15 mmol/L tris buffer solution (pH 7.0). The percentage of the aggregates (1) was 1.77%, the percentage of the aggregates (2) was 1.78%, and the percentage of the monomers was 96.44%.

The antibody solution containing aggregate components (impurities) and monomer components (physiologically active substance of interest) of the antibody proteins was contacted with cation-exchange membrane 21. The amount of the antibody solution added was 20 mL (concentration: 4.74 mg/mL), the flow rate was 1.5 mL/min, and the temperature was 25°C. After the flowing of the antibody solution, cation-exchange membrane 21 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased. The results are shown in Figure 7.

(Reference Comparative Example 1)

In Reference Comparative Example 1, cation-exchange membrane 22 described below was used which had a cation-exchange group density of smaller than 30 mmol/L. Cation-exchange membrane 22 was synthesized as follows: 3.09 g of N-isopropylacrylamide, 1.86 g of butyl methacrylate, and 0.2 g of methacrylic acid were dissolved in 240 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.22 g of a cation-exchange membrane having a graft ratio of 74%. The cation-exchange group density measured in the same way as in Reference Example 1 was 27 mmol/L, which was lower than the density of 30 mmol/L. The mass percentages of cation-exchange group-containing monomers and neutral monomers were 0.017 and 0.983, respectively. The resultant was made into a module (membrane volume: 0.25 mL) to prepare cation-exchange membrane 22 according to Reference Comparative Example 1.
The antibody solution used in Reference Comparative Example 1 was a 15 mmol/L tris buffer solution (pH 7.0). The percentage of the aggregates (1) was 2.27%, the percentage of the aggregates (2) was 1.86%, and the percentage of the monomers was 95.87%.
The antibody solution containing aggregate components (impurities) and monomer components (physiologically active substance of interest) of the antibody proteins was contacted with cation-exchange membrane 22. The amount of the antibody solution added was 20 mL (concentration: 5.13 mg/mL), the flow rate was 1.5 mL/min, and the temperature was 25°C. After the flowing of the antibody solution, cation-exchange membrane 22 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased, but was larger than that in Reference Example 1. The results are shown in Figure 7.

(Reference Comparative Example 2)

In Reference Comparative Example 2, cation-exchange membrane 23 described below was used which had only strong cation-exchange groups having a cation-exchange group density of smaller than 30 mmol/L. Cation-exchange membrane 23 was synthesized as follows: 3.6 g of N-isopropylacrylamide, 1.8 g of butyl methacrylate, and 0.6 g of glycidyl methacrylate were dissolved in 280 mL of an aqueous solution containing 50% by volume of t-butyl alcohol, and the solution was used as a reaction solution after nitrogen bubbling for 30 minutes. 3.00 g (15 cm, 15 filaments) of polyethylene porous hollow fiber having an outer diameter of 3.0 mm, an inner diameter of 2.0 mm, and an average pore size of 0.25 um was placed in a closed container, and the inside air of the container was replaced with nitrogen. Then, the container was cooled with dry ice from outside while irradiated with 200 kGy of $\gamma$ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 5.11 g of a cation-exchange membrane precursor having a graft ratio of 70%.
The hollow fiber containing the graft chain introduced by the radiation graft polymerization method was added to 200 g of a mixed aqueous solution of sodium sulfite and IPA (sodium sulfite/IPA/pure water = 10/15/75 wt%) and reacted at 80°C for 24 hours to convert the epoxy groups in the graft chain to sulfonic acid groups. The hollow fiber thus reacted was washed with pure water. Then, this hollow fiber was added into 0.5 mol/L sulfuric acid and reacted at 80°C for 2 hours to convert the remaining epoxy groups in the graft chain to diol groups. The cation-exchange group density measured in the same way as in Reference Example 1 was 18 mmol/L, which was lower than the density of 30 mmol/L. The mass percentages of cation-exchange group-containing monomers and neutral monomers were 0.03 and 0.97, respectively. The resultant was made into a module (membrane volume: 0.25 mL) to prepare cation-exchange membrane 23 according to Reference Comparative Example 2.
The antibody solution used in Reference Comparative Example 2 was a 15 mmol/L acetate buffer solution (pH 6.0). The percentage of the aggregates (1) was 2.03%, the percentage of the aggregates (2) was 2.06%, and the percentage of the monomers was 95.91%.

The antibody solution containing aggregate components (impurities) and monomer components (physiologically active substance of interest) of the antibody proteins was contacted with cation-exchange membrane 23. The amount of the antibody solution added was 50 mL (concentration: 4.80 mg/mL), the flow rate was 1.5 mL/min, and the temperature was 25°C. After the flowing of the antibody solution, cation-exchange membrane 23 was washed with 10 mL of a 15 mmol/L acetate buffer solution (pH 6.0) of 25°C flowing at a flow rate of 1.5 mL/min. 60 mL of a solution was recovered by the flow-through step and the washing step. The recovered solution was applied to size exclusion chromatography (SEC). As a result, the content of the aggregate components decreased, but was large as compared with Reference Examples, and this aggregate removal performance was insufficient for a large processing volume. The results are shown in Figure 7.

(Reference Comparative Example 3)

In Reference Comparative Example 3, cation-exchange membrane 24 described below was used in which homopolymers were immobilized on the surface of a membrane matrix. Cation-exchange membrane 24 was synthesized as follows: 0.51 g of methacrylic acid was dissolved in 240 mL of an aqueous solution containing 50% by volume of t-butyl alcohol, and the solution was used as a reaction solution after nitrogen bubbling for 30 minutes. 3.0 g (15 cm, 15 filaments) of polyvinylidene fluoride porous hollow fiber having an outer diameter of 2.0 mm, an inner diameter of 1.1 mm, and an average pore size of 0.65 um was placed in a closed container, and the inside air of the container was replaced with nitrogen. Then, the container was cooled with dry ice from outside while irradiated with 25 kGy of γ ray to generate radicals. The polyethylene porous hollow fiber having the obtained radicals was transferred to a glass container, and oxygen in the reaction tube was removed by decreasing the pressure to 200 Pa or lower. 140 mL of the reaction solution adjusted to 40°C was introduced to the container, which was then left standing for 16 hours. Then, the hollow fiber was washed with methanol and dried in vacuum in a vacuum dryer to obtain 3.28 g of a cation-exchange membrane having a graft ratio of 9%. The cation-exchange group density measured in the same way as in Reference Example 1 was 183 mmol/L. The resultant was made into a module (membrane volume: 0.25 mL) to prepare cation-exchange membrane 24 according to Reference Comparative Example 3. The mass percentages of cation-exchange group-containing monomers and neutral monomers were 1.0 and 0, respectively.

The antibody solution used in Reference Comparative Example 3 was a 15 mmol/L tris buffer solution (pH 7.0). The percentage of the aggregates (1) was 2.12%, the percentage of the aggregates (2) was 2.31%, and the percentage of the monomers was 95.57%.

The antibody solution containing aggregate components (impurities) and monomer components (physiologically active substance of interest) of the antibody proteins was contacted with cation-exchange membrane 24. The amount of the antibody solution added was 20 mL (concentration: 5.15 mg/mL), the flow rate was 1.5 mL/min, and the temperature was 25°C. After the flowing of the antibody solution, cation-exchange membrane 24 was washed with 10 mL of a 15 mmol/L tris buffer solution (pH 7.0) of 25°C flowing at a flow rate of 1.5 mL/min. 30 mL of a solution was recovered by the flow-through step and the washing step. The recovered solution was applied to size exclusion chromatography (SEC). As a result, the content of the aggregate components was decreased, but was large as compared with other reference examples. The results are shown in Figure 7.

(Reference Example 30)

Reference Example 30 shows an example in which impurities were reduced by flow-through purification with an anion-exchange chromatographic support media followed by flow-through purification with a cation-exchange chromatographic support media.

(Anion-exchange chromatography step)

In Reference Example 30, Capto Q (GE Healthcare Biosciences Corp.) having a volume of 1 mL was used in the anion-exchange step. The antibody solution was a 15 mmol/L tris buffer solution (pH 7.8). The percentage of the aggregates (1) was 0.42%, the percentage of the aggregates (2) was 1.14%, and the percentage of the monomers was 98.44%. The content of HCP was 306 ppm, and the content of protein A was 3 ppm.

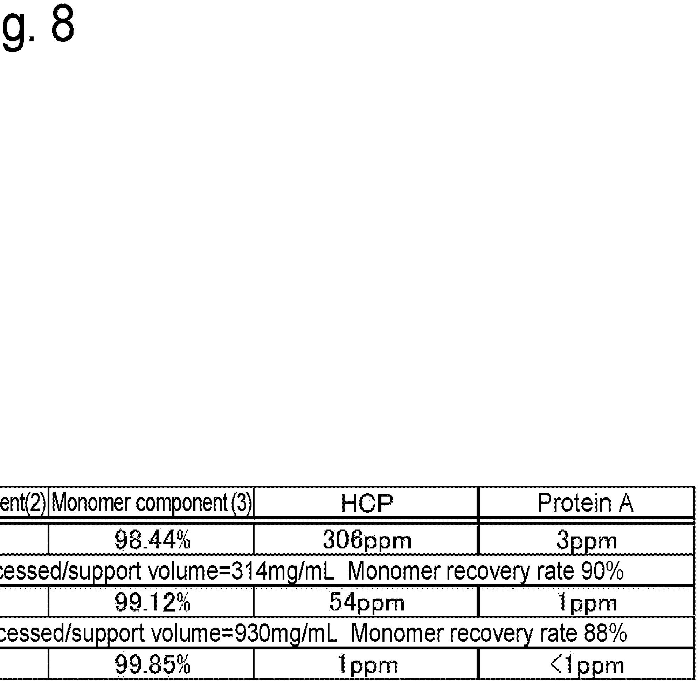

The antibody solution containing aggregate components (impurities) and monomer components (physiologically active substance of interest) of the antibody proteins, HCP, and protein A was contacted with Capto Q having a volume of 1 mL. The amount of the antibody solution added was 50 mL (concentration: 6.28 mg/mL), the flow rate was 1.0 mL/min, and the temperature was 25°C. After the flowing of the antibody solution, Capto Q was washed with 5 mL of a 15 mmol/L tris buffer solution (pH 7.8) of 25°C flowing at a flow rate of 1.0 mL/min. 55 mL of a solution was recovered by the flow-through step and the washing step. The recovered solution was analyzed by size exclusion chromatography (SEC) and ELISA. As a result, the contents of the aggregate components, HCP, and protein A were decreased. The results are shown in Figure 8.

(Cation-exchange chromatography step)

In Reference Example 30, cation-exchange membrane 10 was used in the cation-exchange step. The antibody solution recovered by the anion-exchange step was adjusted to pH 7 by the addition of 0.1 mol/L hydrochloric acid and contacted with cation-exchange membrane 10. The amount of the antibody solution added was 50 mL (concentration: 4.65 mg/mL), the flow rate was 1.5 mL/min, and the temperature was 25°C. After the flowing of the antibody solution, cation-exchange membrane 10 was washed with 10 mL of a 15 mmol/L tris buffer solution (pH 7) of 25°C flowing at a flow rate of 1.5 mL/min. 60 mL of an antibody solution was recovered by the flow-through step and the washing step. The recovered solution was analyzed by size exclusion chromatography (SEC) and ELISA. As a result, the contents of the aggregate components, HCP, and protein A were decreased. The results are shown in Figure 8.

(Reference Example 31)

In Reference Example 31, a culture supernatant containing 0.163 g/L AE6F4 antibodies (human monoclonal antibodies) as antibody proteins was used, and a series of purification steps involving affinity chromatography, anion-exchange chromatography, and cation-exchange chromatography were performed without buffer replacement.

(Affinity chromatography step)

The operation of the affinity chromatography step according to Reference Example 31 was performed at a flow rate of 4 mL/min (300 cm/hr). First, a column packed with 16 mL of Mabselect Sure was equilibrated with 80 mL of a phosphate buffer solution (20 mmol/L sodium phosphate + 150 mmol/L NaCl (pH 8.0)), and 2.5 L of the culture supernatant containing the antibodies was added thereto so that the antibodies were adsorbed onto the column. Next, the column was washed by passing 80 mL of a phosphate buffer solution (20 mmol/L sodium phosphate + 150 mmol/L NaCl (pH 8.0)) and further passing 48 mL of a tris/acetate buffer solution (100 mmol/L (pH 8.0)).
Then, the antibodies were eluted from the column by passing 80 mL of a 25 mmol/L acetate buffer solution (pH 3.4) as an eluting solution. The eluate was adjusted to pH 7.8 by the addition of a 1 mol/L tris buffer solution to obtain an antibody solution. The obtained antibody solution had an electric conductivity of 1.8 mS/cm. The obtained antibody solution was mixed with an antibody solution having the same solution composition thereas and containing more aggregates to prepare an antibody solution for use in the anion-exchange chromatography step mentioned later. In the antibody solution, the percentage of the aggregates (1) was 0.83%, the percentage of the aggregates (2) was 1.12%, and the percentage of the monomers was 98.05%. The content of HCP was 317 ppm, and the content of protein A was 3 ppm.

(Anion-exchange chromatography step)

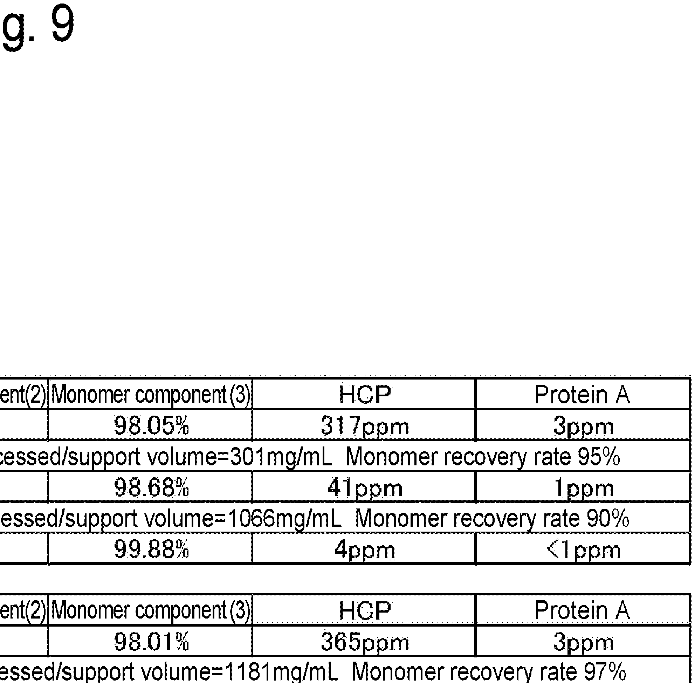

The antibody solution was purified in the anion-exchange step according to Reference Example 31 using Capto Q (GE Healthcare Biosciences Corp.) having a volume of 1 mL. The amount of the antibody solution added to Capto Q was 81 mL (concentration: 3.72 mg/mL), the flow rate was 1.0 mL/min, and the temperature was 25°C. After the flowing of the antibody solution in Capto Q, Capto Q was washed by passing 5 mL of a buffer (pH 7.8) having the same composition as that for the antibody solution at a flow rate of 1.0 mL/min. A total of 86 mL of a solution was recovered by the flow-through step and the washing step. The recovered solution was analyzed by size exclusion chromatography (SEC) and ELISA. As a result, the contents of the aggregate components, HCP, and protein A were decreased. The results are shown in Figure 9.

(Cation-exchange chromatography step)

Cation-exchange membrane 10 was used in the cation-exchange chromatography step according to Reference Example 31. The antibody solution recovered by the anion-exchange step was adjusted to pH 7.0 by the addition of acetic acid. The resulting antibody solution had an electric conductivity of 1.9 mS/cm. Then, the antibody solution was contacted with cation-exchange membrane 10. The amount of the antibody solution added was 80 mL (concentration: 3.33 mg/mL), the flow rate was 1.5 mL/min, and the temperature was 25°C. After the flowing of the antibody solution in cation-exchange membrane 10, cation-exchange membrane 10 was washed by passing 10 mL of a buffer (pH 7.0) having the same composition as that for the antibody solution at a flow rate of 1.5 mL/min. A total of 90 mL of a solution was recovered by the flow-through step and the washing step. The recovered solution was analyzed by size exclusion chromatography (SEC) and ELISA. As a result, the contents of the aggregate components, HCP, and protein A were decreased. The results are shown in Figure 9.

(Reference Example 32)

In Reference Example 32, a culture supernatant containing 0.163 g/L AE6F4 antibodies (human monoclonal antibodies) as antibody proteins was used, and a series of purification steps involving affinity chromatography, anion-exchange chromatography, and cation-exchange chromatography were performed without buffer replacement.

(Affinity chromatography step)

The operation of the affinity chromatography step according to Reference Example 32 was performed at a flow rate of 4 mL/min (300 cm/hr). First, a column packed with 16 mL of Mabselect Sure was equilibrated with 80 mL of a phosphate buffer solution (20 mmol/L sodium phosphate + 150 mmol/L NaCl (pH 8.0)), and 2.5 L of the culture supernatant containing the antibodies was added thereto so that the antibodies were adsorbed onto the column. Next, the column was washed by passing 80 mL of a phosphate buffer solution (20 mmol/L sodium phosphate + 150 mmol/L NaCl (pH 8.0)) and further passing 48 mL of a tris/acetate buffer solution (100 mmol/L (pH 8.0)).
Then, the antibodies were eluted from the column by passing 80 mL of a 25 mmol/L acetate buffer solution (pH 3.4) as an eluting solution. The eluate was adjusted to pH 7.8 by the addition of a 1 mol/L tris buffer solution to obtain an antibody solution. The electric conductivity of the obtained antibody solution was 1.8 mS/cm. The obtained antibody solution was mixed with an antibody solution having the same solution composition thereas and containing more aggregates to prepare an antibody solution for use in the anion-exchange chromatography step mentioned later. In the antibody solution, the percentage of the aggregates (1) was 0.78%, the percentage of the aggregates (2) was 1.21%, and the percentage of the monomers was 98.01%. The content of HCP was 365 ppm, and the content of protein A was 3 ppm.

(Anion-exchange chromatography step)

QyuSpeed D (Asahi Kasei Medical Co., Ltd.) having a volume of 0.25 mL was used in the anion-exchange step according to Reference Example 32. The amount of the antibody solution added to QyuSpeed D was 80 mL (concentration: 3.69 mg/mL), the flow rate was 1.5 mL/min, and the temperature was 25°C. After the flowing of the antibody solution in QyuSpeed D, QyuSpeed D was washed by passing 10 mL of a buffer (pH 7.8) having the same composition as that for the antibody solution at a flow rate of 1.5 mL/min. A total of 90 mL of a solution was recovered by the flow-through step and the washing step. The recovered solution was analyzed by size exclusion chromatography (SEC) and ELISA. As a result, the contents of the aggregate components, HCP, and protein A were decreased. The results are shown in Figure 9.

(Cation-exchange chromatography step)

Cation-exchange membrane 10 was used in the cation-exchange chromatography step according to Reference Example 32. The antibody solution recovered by the anion-exchange step was adjusted to pH 7.0 by the addition of acetic acid. The resulting antibody solution had an electric conductivity of 1.9 mS/cm. Then, the antibody solution was contacted with cation-exchange membrane 10. The amount of the antibody solution added was 80 mL (concentration: 3.17 mg/mL), the flow rate was 1.5 mL/min, and the temperature was 25°C. After the flowing of the antibody solution, cation-exchange membrane 10 was washed by passing 10 mL of a buffer (pH 7.0) having the same composition as that for the antibody solution at a flow rate of 1.5 mL/min. A total of 90 mL of a solution was recovered by the flow-through step and the washing step. The recovered solution was analyzed by size exclusion chromatography (SEC) and ELISA. As a result, the contents of the aggregate components, HCP, and protein A were decreased. The results are shown in Figure 9.

(Example 1)

(Preparation of buffer)

A solution consisting of 0.5451 g of Tris (molecular weight: 121.14), 3 mL of 1 mol/L NaCl, and water was adjusted to pH 7.0 with 0.1 N HCl and further brought up to 300 mL with water to obtain a buffer consisting of 15 mmol/L Tris-HCl (pH 7.0) and 10 mmol/L NaCl.

(Preparation of purified solution of antibody monomer)

AE6F4 antibodies purified with a protein A column in the same way as in Reference Example 1 were prepared.

A solution containing the antibodies was buffer-replaced with the aforementioned buffer using an ultrafiltration membrane (Amicon(R) Ultra-15, centrifugal filter unit, Merck Millipore) having a molecular weight cutoff value of 30000. The obtained solution containing the antibodies had an antibody concentration of 5 mg/mL. The filtrate from the ultrafiltration membrane was further suction-filtered through a filter having a pore size of 0.20 $\mu$m (Thermo Scientific(R) Nalgene(R) Rapid-Flow(R) PES membrane filter unit) to obtain a purified solution of antibody monomers.

(Preparation of solution containing antibody aggregate)

A portion of the obtained purified solution of antibody monomers was adjusted to pH 3.0 with 1 mol/L hydrochloric acid (HCl) and left standing at room temperature for 1 hour to form antibody aggregates. Next, the solution was adjusted to pH 5.0 or higher with 1 mol/L sodium hydroxide (NaOH). The solution containing the formed antibody aggregates was buffer-replaced with the aforementioned buffer using an ultrafiltration membrane (Amicon(R) Ultra-15, centrifugal filter unit, Merck Millipore) having a molecular weight cutoff value of 30000. The filtrate from the ultrafiltration membrane was further suction-filtered through a filter having a pore size of 0.20 $\mu$m (Thermo Scientific(R) Nalgene(R) Rapid-Flow(R) PES membrane filter unit) to obtain a solution containing antibody aggregates.

(Preparation of solution containing antibody monomer and aggregate)

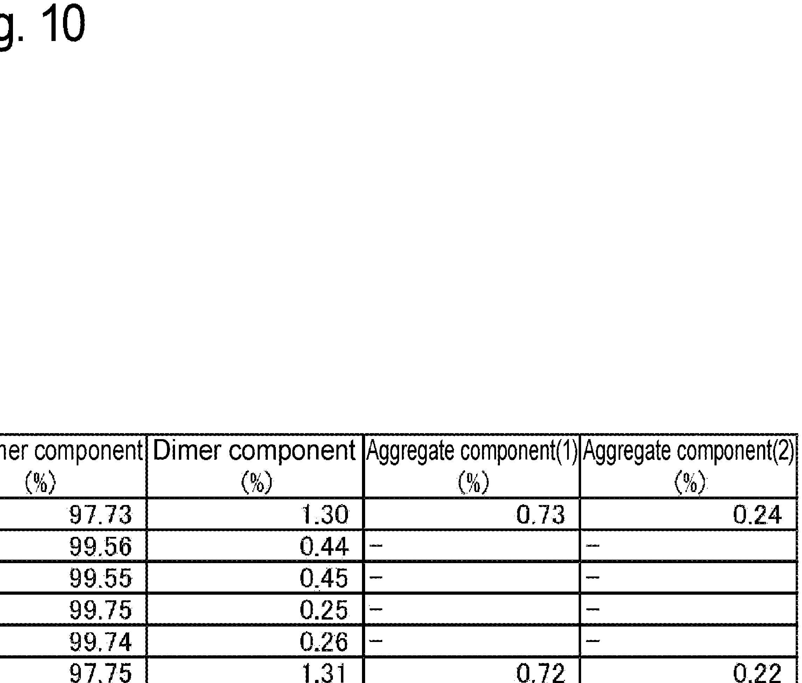

150 mL of the purified solution of antibody monomers was mixed with 30 mL of the solution containing antibody aggregates. Then, this mixed solution was suction-filtered through a 0.20 $\mu$m filter (Thermo Scientific(R) Nalgene(R) Rapid-Flow(R) PES membrane filter unit) and further filtered through a 0.10 $\mu$m filter (Merck Millipore, Millipak 20 filter unit) at a filtration pressure of 19.6 kPa to obtain a solution containing antibody monomers and aggregates. In the solution containing antibody monomers and aggregates, as shown in Figure 10, the percentage of antibody monomers was 97.73%, the percentage of antibody dimers was 1.30%, and the percentage of antibody trimer or higher aggregates was 0.97 (= 0.73 + 0.24) %.

(Pre-filtration)

Cation-exchange membrane 1 according to Reference Example 1 was provided. The cation-exchange membrane was washed with 30 mL of a buffer in a flow volume of 1.5 mL/min. Next, 78 mL of the solution containing antibody monomers and aggregates was flow-through filtered by flowing through the cation-exchange membrane in a flow volume of 1.5 mL/min. The total amount of antibodies flowing per mL of the membrane volume was 1.5 g. Then, antibodies remaining on the cation-exchange membrane were recovered using 10 mL of a buffer flowing through the cation-exchange membrane in a flow volume of 1.5 mL/min. The filtrate obtained by the flow-through filtration and the recovered solution of antibodies recovered using a flowing buffer were combined to prepare a solution after the pre-filtration. As shown in Figure 10, in the solution after the pre-filtration, the percentage of antibody monomers was 99.56%, the percentage of antibody dimers was 0.44%, and antibody trimer or higher aggregates were not detected. Accordingly, antibody dimer or higher aggregates were remarkably removed.

(Main filtration)

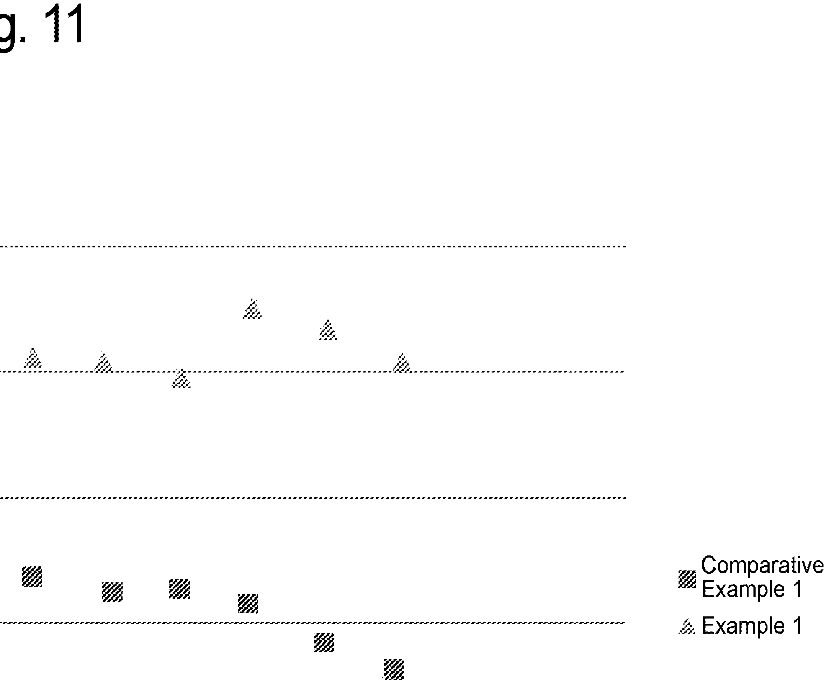

A virus removal filter (Planova(R) 20N, Asahi Kasei Medical Co., Ltd.) having regenerated cellulose hollow fiber based on the copper ammonia method was prepared. This virus removal filter comprises a primary-side surface to which the solution after the pre-filtration is to be applied, and a secondary-side surface facing the primary-side surface. The pore size decreases and in turn increases from the primary side toward the secondary side on the cross section of the virus removal filter. 30 mL of the solution obtained by the pre-filtration was immediately filtered through the virus removal filter at a filtration pressure of 0.8 kgf/cm$^2$ without being processed. The filtrate was recovered as 15 fractions (2 mL each) and used as a solution after the main filtration. As shown in Figure 10, in the solution after the main filtration, the percentage of monomers was 99.55%, the percentage of antibody dimers was 0.45%, and antibody trimer or higher aggregates were not detected. As shown in Figure 11, no reduction in permeate flux (Flux) was observed in the main filtration.

(Reference Example 33)

The purified solution of antibody monomers prepared in Example 1 was suction-filtered through a 0.20 $\mu$m filter and further filtered through a 0.10 $\mu$m filter at a filtration pressure of 19.6 kPa. In the purified solution of antibody monomers filtered through the 0.20 $\mu$m filter and the 0.10 $\mu$m filter, as shown in Figure 10, the percentage of antibody monomers was 99.75%, the percentage of antibody dimers was 0.25%, and antibody trimer or higher aggregates were not detected.

Then, 30 mL of the purified solution of antibody monomers was filtered through a virus removal filter (Planova(R) 20N, Asahi Kasei Medical Co., Ltd.) at a filtration pressure of 78.4 kPa. The filtrate was recovered as 15 fractions (2 mL each). As shown in Figure 10, in the virus-removed purified solution of antibody monomers, the percentage of antibody monomers was 99.74%, the percentage of antibody dimers was 0.26%, and antibody trimer or higher aggregates were not detected.

(Comparative Example 1)

30 mL of the solution containing antibody monomers and aggregates, prepared in Example 1 was filtered through a virus removal filter (Planova(R) 20N, Asahi Kasei Medical Co., Ltd.) at a filtration pressure of 78.4 kPa. The filtrate was recovered as 15 fractions (2 mL each). As shown in Figure 10, in the virus-removed purified solution of antibody monomers, the percentage of antibody monomers was 99.75%, the percentage of antibody dimers was 1.31%, and the percentage of antibody trimer or higher aggregates was 0.94 (= 0.72 + 0.22) %. As shown in Figure 11, reduction in permeate flux (Flux) was observed in the filtration step using the virus removal membrane.

(Reference Comparative Example 4)

A cation-exchange membrane according to Comparative Example 2 containing a strong cation-exchange group (sulfonic acid group) and containing no weak cation-exchange group was prepared according to Example 1 of National Publication of International Patent Application No. 2012-519065. Next, the cation-exchange membrane was washed with 12 mL of the buffer prepared in Example 1 in a flow volume of 0.6 mL/min. Next, 33 mL of the solution containing antibody monomers and aggregates was flow-through filtered by flowing through the cation-exchange membrane in a flow volume of 1.5 mL/min. Then, antibodies remaining on the cation-exchange membrane were recovered using 4 mL of a buffer flowing through the cation-exchange membrane in a flow volume of 1.5 mL/min. The filtrate obtained by the flow-through filtration and the recovered solution of antibodies recovered using a flowing buffer were combined to prepare a solution after the pre-filtration. As shown in Figure 10, in the solution after the pre-filtration, the percentage of antibody monomers was 98.99%, and the percentage of antibody dimers was 1.01%. Accordingly, the percentage of antibody dimers was higher than that of Example 1.

**Claims**

1.   A method for purifying a protein, comprising:

providing a solution containing a monomer and aggregates of the protein of interest;
a purification step of removing the aggregates of the protein of interest using a cation-exchange chromatographic support media to obtain a purified solution of the monomer, the cation-exchange chromatographic support media comprising at least one type of weak cation-exchange group and having a cation-exchange group density higher than 30 mmol/L; and
a virus removal step of removing viruses from the purified solution using a virus removal membrane having a virus logarithmic reduction value of 3 or more.

2.   The method for purifying a protein according to claim 1, wherein the cation-exchange chromatographic support media comprises a membrane matrix and a copolymer immobilized on the surface of the membrane matrix, and the copolymer comprises a (meth)acrylamide-based compound and/or a (meth)acrylate-based compound as monomer units.

3.   The method for purifying a protein according to claim 2, wherein monomer units other than monomer units having cation-exchange groups in the copolymer are neutral monomers having no charge, and the neutral monomers are a hydrophobic monomer unit and/or a hydrophilic monomer unit.

4.   The method for purifying a protein according to claim 3, wherein the hydrophobic monomer unit has a linear or branched alkyl group having four or more carbon atoms.

5.   The method for purifying a protein according to claim 3 or 4, wherein the mass percentage of the hydrophobic monomer unit and/or the hydrophilic monomer unit in the copolymer is higher than that of the monomer units having cation-exchange groups.

6.   The method for purifying a protein according to any of claims 1 to 5, wherein the weak cation-exchange group is derived from any of an acrylic acid monomer, a methacrylic acid monomer, an acrylic acid compound monomer, and a methacrylic acid compound monomer.

7. The method for purifying a protein according to any one of claims 1 to 6, wherein the cation-exchange groups comprised in the cation-exchange chromatographic support media are only weak cation-exchange groups.

8. The method for purifying a protein according to any one of claims 1 to 6, wherein the cation-exchange groups comprised in the cation-exchange chromatographic support media include a weak cation-exchange group and a strong cation-exchange group.

9. The method for purifying a protein according to claim 8, wherein the strong cation-exchange group is a sulfonic acid group.

10. The method for purifying a protein according to any one of claims 2 to 5, wherein the copolymer is immobilized on the surface of the membrane matrix through a covalent bond.

11. The method for purifying a protein according to any one of claims 3 to 5, wherein the hydrophilic monomer unit comprises at least one of isopropylacrylamide and 2-hydroxyethyl methacrylate.

12. The method for purifying a protein according to any one of claims 2 to 5, wherein the membrane matrix comprises polyethylene.

13. The method for purifying a protein according to claim 12, wherein the graft ratio of the copolymer graft-polymerized onto the membrane matrix is 20 to 200%.

14. The method for purifying a protein according to any one of claims 2 to 5, wherein the membrane matrix comprises polyvinylidene fluoride.

15. The method for purifying a protein according to claim 14, wherein the graft ratio of the copolymer graft-polymerized onto the membrane matrix is 5 to 100%.

16. The method for purifying a protein according to any one of claims 2 to 5, wherein the copolymer substantially has no cross-linked structure.

17. The method for purifying a protein according to any one of claims 2 to 5, wherein the copolymer comprises a monomer unit containing two or more polymerizable functional groups.

18. The method for purifying a protein according to any one of claims 1 to 17, wherein the cation-exchange group density is higher than 45 mmol/L.

19. The method for purifying a protein according to any one of claims 1 to 18, wherein the virus removal membrane comprises a primary-side surface to which the purified solution of the monomer is to be applied, and a secondary-side surface facing the primary-side surface, wherein
the virus removal membrane comprises at least a site where a pore size decreases from the primary side toward the secondary side on the cross section of the virus removal membrane.

20. The method for purifying a protein according to any one of claims 1 to 18, wherein the virus removal membrane comprises a primary-side surface to which the purified solution of the monomer is to be applied, and a secondary-side surface facing the primary-side surface, wherein
a pore size decreases and in turn increases from the primary side toward the secondary side on the cross section of the virus removal membrane.

21. The method for purifying a protein according to any one of claims 1 to 18, wherein the virus removal membrane comprises a primary-side surface to which the purified solution of the monomer is to be applied, and a secondary-side surface facing the primary-side surface, wherein
a pore size decreases and in turn becomes constant from the primary side toward the secondary side on the cross section of the virus removal membrane, and a most closely packed layer is comprised near the secondary-side surface.

**22.** The method for purifying a protein according to any one of claims 1 to 20, wherein the virus removal membrane comprises cellulose.

**23.** The method for purifying a protein according to any one of claims 1 to 20, wherein the virus removal membrane comprises a hydrophilized synthetic polymer.

**24.** The method for purifying a protein according to any one of claims 1 to 23, wherein no additional step is comprised between the purification step and the virus removal step.

**25.** The method for purifying a protein according to any one of claims 1 to 24, wherein the purification step and the virus removal step are continuously performed.

**26.** The method for purifying a protein according to any one of claims 1 to 23, wherein the protein of interest is an antibody.

**27.** The method for purifying a protein according to claim 26, wherein the antibody is a monoclonal antibody.

**28.** The method for purifying a protein according to any one of claims 1 to 23, wherein the protein of interest is a recombinant protein.

# Fig. 1

Film thickness

# Fig. 2

# Fig. 3

# Fig. 4

1

# Fig. 5

250000

200000

150000

# Fig. 6

# g. 7

| After processing (content) | | | Monomer recovery rate | Reduction rate of percentage of aggregate |
|---|---|---|---|---|
| ate component(1) | Aggregate component(2) | Monomer component(3) | | |
| 0.04% | 0.29% | 99.67% | 94% | 91% |
| 0.03% | 0.22% | 99.72% | 80% | 91% |
| 0.06% | 0.60% | 99.35% | 84% | 81% |
| 0.46% | 1.27% | 98.27% | 90% | 70% |
| 0.09% | 0.40% | 99.51% | 90% | 91% |
| 0.17% | 0.35% | 99.47% | 84% | 87% |
| 0.00% | 0.26% | 99.74% | 91% | 93% |
| 0.02% | 0.26% | 99.72% | 89% | 95% |
| 0.00% | 0.34% | 99.66% | 89% | 93% |
| 0.00% | 0.25% | 99.75% | 93% | 87% |
| 0.11% | 0.41% | 99.49% | 90% | 84% |
| 0.24% | 0.99% | 98.77% | 90% | 77% |
| 0.13% | 0.39% | 99.48% | 92% | 87% |
| 0.37% | 1.01% | 98.62% | 89% | 70% |
| 0.17% | 0.58% | 99.25% | 88% | 77% |
| 0.06% | 0.87% | 99.07% | 90% | 75% |
| 0.11% | 0.71% | 99.18% | 93% | 75% |
| 0.13% | 0.48% | 99.39% | 88% | 86% |
| 0.28% | 0.57% | 99.15% | 88% | 84% |

# g. 8

| ent(2) | Monomer component (3) | HCP | Protein A |
|---|---|---|---|
| | 98.44% | 306ppm | 3ppm |
| essed/support volume=314mg/mL  Monomer recovery rate 90% | | | |
| | 99.12% | 54ppm | 1ppm |
| essed/support volume=930mg/mL  Monomer recovery rate 88% | | | |
| | 99.85% | 1ppm | <1ppm |

EP 3 406 623 A1

# g. 9

EP 3 406 623 A1

| ent(2) | Monomer component (3) | HCP | Protein A |
|---|---|---|---|
| | 98.05% | 317ppm | 3ppm |
| essed/support volume=301mg/mL  Monomer recovery rate 95% | | | |
| | 98.68% | 41ppm | 1ppm |
| essed/support volume=1066mg/mL  Monomer recovery rate 90% | | | |
| | 99.88% | 4ppm | <1ppm |

| ent(2) | Monomer component (3) | HCP | Protein A |
|---|---|---|---|
| | 98.01% | 365ppm | 3ppm |
| essed/support volume=1181mg/mL  Monomer recovery rate 97% | | | |

Fig. 10

| ...er component (%) | Dimer component (%) | Aggregate component(1) (%) | Aggregate component(2) (%) |
| --- | --- | --- | --- |
| 97.73 | 1.30 | 0.73 | 0.24 |
| 99.56 | 0.44 | – | – |
| 99.55 | 0.45 | – | – |
| 99.75 | 0.25 | – | – |
| 99.74 | 0.26 | – | – |
| 97.75 | 1.31 | 0.72 | 0.22 |

Fig. 11

Comparative Example 1

Example 1

**INTERNATIONAL SEARCH REPORT**

| International application No. |
| --- |
| PCT/JP2017/001364 |

A. CLASSIFICATION OF SUBJECT MATTER

*C07K1/18*(2006.01)i, *B01D15/36*(2006.01)i, *B01J20/281*(2006.01)i, *G01N30/00*(2006.01)i, *G01N30/88*(2006.01)i

According to International Patent Classification (IPC) or to both national classification and IPC

B. FIELDS SEARCHED

Minimum documentation searched (classification system followed by classification symbols)
C07K1/18, B01D15/36, B01J20/281, G01N30/00, G01N30/88

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2013-189427 A (EMD Millipore Corp.),<br>26 September 2013 (26.09.2013),<br>claims; examples 9 to 12; fig. 11<br>& US 2013/0245139 A1 & WO 2013/138098 A1<br>claims; examples 9 to 12; fig. 11<br>& EP 2639239 A2 & KR 10-2013-0105340 A<br>& CN 103382215 A | 1-17,23-28<br>1-28 |
| Y | WO 2014/061411 A1 (JNC Corp.),<br>24 April 2014 (24.04.2014),<br>claims; examples 7, 8<br>& US 2015/0266919 A1<br>claims; examples 7, 8<br>& WO 2014/061411 A & EP 2910943 A1<br>& CN 104718450 A | 1-28 |

| ☒ Further documents are listed in the continuation of Box C. | ☐ See patent family annex. |
| --- | --- |

| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |

| Date of the actual completion of the international search<br>24 February 2017 (24.02.17) | Date of mailing of the international search report<br>07 March 2017 (07.03.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2017/001364

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2015/156401 A1  (Asahi Kasei Medical Co., Ltd.), 15 October 2015 (15.10.2015), claims; paragraphs [0023], [0075] to [0090] & KR 10-2016-0087892 A  & CN 105980038 A | 19-28 |
| A | Data Sheet, Viresolve(registered trademark) Pro Solution, Merck Ltd., [online], [retrieval date:24 February 2017 (24.02.2017)], [Retrieved from the internet:<URL:http://www.merckmillipore.com/JP/ja/products/biopharmaceutical-manufacturing/downstream-processing/virus-safety/virus-filtration/702.qB.6KkAAAFAU.BkiQpx,nav> | 1-28 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4776615 B
- JP 5234727 B
- JP 2013189427 A
- WO 2003026779 A
- WO 2004035180 A
- WO 2015156401 A
- WO 2015156403 A
- WO 2012519065 A

**Non-patent literature cited in the description**

- *Proceedings of Annual Meeting of The Society for Biotechnology,* 1994, vol. 65, 65